# EUROPÄISCHE PATENTSCHRIFT

(11) **EP 2 883 528 B1**
(45) Veröffentlichungstag und Bekanntmachung des Hinweises auf die Patenterteilung: **31.01.2018**
(21) Anmeldenummer: 13197311.7
(22) Anmeldetag: 15.12.2013
(51) Int. Cl.: A61K 6/087, C07D 251/54, C07D 493/10, C08G 73/06

(54) **Dentalwerkstoffe auf Basis von polymerisierbaren Aziden und Alkinen**
Dental materials based on polymerisable azides and alkynes
Matériaux dentaires à base d'alcynes et d'azides polymérisables

(43) Veröffentlichungstag der Anmeldung: 17.06.2015
(73) Patentinhaber: Ivoclar Vivadent AG, 9494 Schaan (LI)
(72) Erfinder: Moszner, Norbert, 9493 Mauren (LI); Burtscher, Peter, 6830 Rankweil (AT); Schubert, Ulrich S., 07743 Jena (DE); Hager, Martin, 07743 Jena (DE); Happ, Bobby, 07745 Jena (DE); Sandmann, Benedict, 07743 Jena (DE)
(74) Vertreter: Uexküll & Stolberg

(56) Entgegenhaltungen:
- WO-A1-2006/012569
- WO-A2-2013/034777
- WEI, Q. ET AL.: "Efficient Polymerization of Azide and Active Internal Alkynes", MACROMOLECULAR: RAPID COMMUNICATIONS., Bd. 33, Nr. 16, 2012, Seiten 1356-1361, XP002723971, WILEY VCH VERLAG, WEINHEIM; DE ISSN: 1022-1336, DOI: 10.1002/marc.201200212

## Beschreibung

Die vorliegende Erfindung betrifft thermisch härtende Dentalwerkstoffe und insbesondere dentale Kompositmaterialien mit hervorragenden mechanischen Eigenschaften zur Herstellung von dentalen Kompositen für Inlays, Onlays, Kronen, Brücken oder Verblendmaterialien.

Dentale Komposite, die z.B. als direktes Füllungsmaterial, Inlay, Onlay, Kronen oder Verblendwerkstoff eingesetzt werden, bestehen in der Regel aus einer polymerisierbaren organischen Matrix und aus einem oder mehreren Füllstoffen. Je nach Art der Füllstoffe, der Monomermatrix und der Anwendung kann der Füllgrad zwischen ca. 50-90 Gew.-% variieren. Die Komposite werden üblicherweise nach der Partikelgrösse und Zusammensetzung der Füllstoffe unterteilt in Makrofüller-Komposite, homogene und heterogene Mikrofüller-Komposite und Hybrid-Komposite (F. Lutz, R. W. Phillips, A classification and evaluation of composite resin systems, J. Prosthet. Dent. 50 (1983) 480-488).

Die polymerisierbare organische Matrix besteht üblicherweise vor allem aus einer Mischung von Monomeren, Initiator-Komponenten, Stabilisatoren und Pigmenten (J. Viohl, K. Dermann, D. Quast, S. Venz, Die Chemie zahnärztlicher Füllungskunststoffe, Carl Hanser Verlag, München-Wien 1986, 21-27). Dabei werden als Harze meist Mischungen von Dimethacrylaten eingesetzt (vgl. A. Peutzfeldt, Resin composites in dentistry: the monomer systems, Eur. J. Oral. Sci. 1997, 105, 97-116; J. W. Nicolson, H. M. Anstice, The chemistry of modern dental filling materials, J. Chem Ed. 1999, 76, 1497-1501; J. W. Stansburry, Curing dental resins and composites by photopolymerization, J. Esthet. Dent. 2000, 12, 300-308; N. Moszner, T. Hirt, New Polymer-Chemical Developments in Clinical Dental Polymer Materials: Enamel-Dentin Adhesives and Restorative Composites, J. Polym. Sci. Part A: Polym. Chem. 2012, 50, 4369-4402). Beispiele hierfür sind die hochviskosen Dimethacrylate 2,2-Bis[4-(2-hydroxy-3-methacryloyloxypropyl)phenyl]-propan (Bis-GMA) und 1,6-Bis-[2-methacryloyloxyethoxycarbonylamino]-2,4,4-trimethylhexan (UDMA) und die als Verdünnermonomere genutzten niedriger viskosen Dimethacrylate wie Bismethacryloyloxymethyltri-cyclo[5.2.1]decan (TCDMA), Decandiol-1,10-dimethacrylate (D₃MA) und Triethylenglycoldimethacrylat (TEGDMA).

Die Aushärtung der bekannten Dimethacrylat-basierenden dentalen Komposite kann durch thermische, redoxinitiierte oder lichtinduzierte radikalische Polymerisation unter Verwendung geeigneter Initiatoren erfolgen. Es hat sich allerdings als nachteilig herausgestellt, dass es bei diesen Kompositen meist schon innerhalb von Sekunden und bei entsprechend geringem Monomerumsatz am sogenannten Gelpunkt zur Bildung eines Polymernetzwerks kommt, in dem Füllerpartikel fest eingebunden sind. Das fertige 3-dimensionale Polymernetzwerk enthält auch bei nahezu vollständigem Monomerumsatz zahlreiche nichtumgesetzte Doppelbindungen. Daher sind die mit den bekannten Dimethacrylat-basierenden dentalen Kompositen erzielbaren Vernetzungsdichten und somit auch die erreichbaren mechanischen Eigenschaften stark begrenzt.

Der Erfindung liegt daher die Aufgabe zugrunde, Dentalwerkstoffe bereitzustellen, die sich durch höheren Umsatz der polyreaktionsfähigen Gruppen, verbesserte mechanische Eigenschaften und vor allem durch einen erhöhten Elastizitätsmodul auszeichnen und sich insbesondere zur Herstellung von dentalen Kompositen für Inlays, Onlays, Kronen, Brücken oder Verblendmaterialien eignen. Vorzugsweise sollen diese Dentalwerkstoffe ohne Metallkatalysator und in kontrollierter Weise ohne übermässige Wärmeentwicklung aushärtbar sein.

Diese Aufgabe wird erfindungsgemäss gelöst durch die Verwendung eines Dentalwerkstoffs, der mindestens eine Verbindung der Formel I oder I' enthält 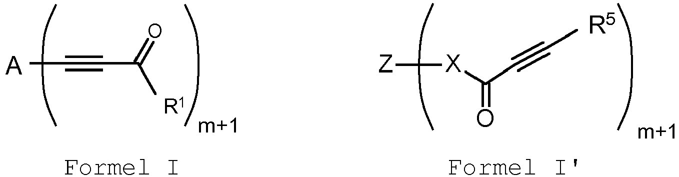 in denen
- A: jeweils unabhängig für einen (m+1)-wertigen Phenylenrest steht,
- R¹: jeweils unabhängig für einen aliphatischen linearen oder verzweigten C₁-C₂₀-Alkyl-Rest, der durch -O- oder -S- unterbrochen sein kann, für einen Phenylrest, der einen oder mehrere Substituenten tragen kann, die vorzugsweise unabhängig voneinander aus der Gruppe bestehend aus CH₃, C₂H₅, OH und OCH₃ ausgewählt sind, oder für R³ steht,
- R²: jeweils unabhängig für H, einen aliphatischen linearen oder verzweigten C₁-C₂₀-Alkyl-Rest, der durch -O- oder -S- unterbrochen sein kann, oder für einen Phenylrest steht, der einen oder mehrere Substituenten tragen kann, die vorzugsweise unabhängig voneinander aus der Gruppe bestehend aus CH₃, C₂H₅, OH und OCH₃ ausgewählt sind,
- R³: jeweils unabhängig für -Z-Y-R² oder -Z-C(=O)-≡-A(R⁴)ₘ steht,
- R⁴: jeweils unabhängig für -≡-C(=O)-R³ steht,
- R⁵: jeweils unabhängig für Phenyl oder R⁶ steht,
- R⁶: jeweils unabhängig für -C(=O)-X-R² oder -C(=O)-X-Z(R⁷)m steht,
- R⁷: jeweils unabhängig für -X-C(=O)-≡-R⁶ steht,
- X: jeweils unabhängig für O, S, NH oder CH₂ steht, wobei X entfällt, wenn R⁵ für Phenyl steht,
- Y: jeweils unabhängig für -C(O)-O-, -O-C(O)-, O oder S steht,
- Z: für einen (m+1)-wertigen aliphatischen linearen oder verzweigten C₁-C₂₀-Alkylen-Rest, der durch -O- oder -S- unterbrochen sein kann, oder für einen Phenylenrest steht, der ein oder mehrere Substituenten tragen kann, die vorzugsweise unabhängig voneinander aus der Gruppe bestehend aus CH₃, C₂H₅, OH und OCH₃ ausgewählt sind, und
- m: jeweils unabhängig die Werte 1 bis 5 annehmen kann,
zur Herstellung von dentalen Kompositen oder dentalen Restaurationsmaterialien.

Der Hinweis, dass ein Rest durch eine Gruppe wie beispielsweise -O-unterbrochen sein kann, ist so zu verstehen, dass die Gruppe in die Kohlenstoffkette des Restes eingeschoben wird, d.h. beidseitig von Kohlenstoffatomen begrenzt wird. Die Anzahl dieser Gruppen ist daher um mindestens 1 kleiner als die Zahl der Kohlenstoffatome, und die Gruppen können nicht endständig sein. Erfindungsgemäss sind Reste, die nicht durch die genannten Gruppen unterbrochen sind, bevorzugt.

Erfindungsgemäss werden nur solche Verbindungen in Erwägung gezogen, die in mit der chemischen Valenzlehre vereinbar sind.

Besonders bevorzugt sind solche Verbindungen der Formeln I und I', bei denen jeweils unabhängig voneinander
- A: jeweils unabhängig für einen (m+1)-wertigen Phenylenrest steht,
- R¹: jeweils unabhängig für einen aliphatischen linearen oder verzweigten C₁-C₁₀-Alkyl-Rest, insbesondere C₁-C₆-Alkyl-Rest und vorzugsweise C₁-C₃-Alkyl-Rest, der durch -O- oder -S-unterbrochen sein kann, oder für R³ steht,
- R²: jeweils unabhängig für H, einen aliphatischen linearen oder verzweigten C₁-C₁₀-Alkyl-Rest, insbesondere C₁-C₆-Alkyl-Rest und vorzugsweise C₁-C₃-Alkyl-Rest steht, der durch -O- oder -S- unterbrochen sein kann,
- R³: jeweils unabhängig für -Z-Y-R² oder -Z-C(=O)-≡-A(R⁴)ₘ steht,
- R⁴: jeweils unabhängig für -≡-C(=O)-R³ steht,
- R⁵: jeweils unabhängig für Phenyl oder R⁶ steht,
- R⁶: jeweils unabhängig für -C(=O)-X-R² oder -C(=O)-X-Z(R⁷)ₘ steht,
- R⁷: jeweils unabhängig für -X-C(=O)-≡-R⁶ steht,
- X: jeweils unabhängig für O, S, NH oder CH₂ steht, wobei X entfällt, wenn R⁵ für Phenyl steht,
- Y: jeweils unabhängig für -C(O)-O- oder -O-C(O)- steht,
- Z: für einen (m+1)-wertigen aliphatischen linearen oder verzweigten C₁-C₁₀-Alkylen-Rest, insbesondere C₁-C₆-Alkylen-Rest und vorzugsweise C₁-C₃-Alkylen-Rest, der durch -O- oder -S- unterbrochen sein kann, oder für einen Phenylenrest steht, der ein oder mehrere Substituenten tragen kann, die vorzugsweise unabhängig voneinander aus der Gruppe bestehend aus CH₃, C₂H₅, OH und OCH₃ ausgewählt sind, und
- m: jeweils unabhängig die Werte 1, 2 oder 3 annehmen kann.

Besonders bevorzugt sind dabei Verbindungen, in denen alle Variablen eine der oben definierten Bedeutungen und insbesondere eine der bevorzugten Bedeutungen haben.

In einer bevorzugten Ausführungsform enthält der Dentalwerkstoff mindestens eine Verbindung mit einer der Formeln IA bis ID 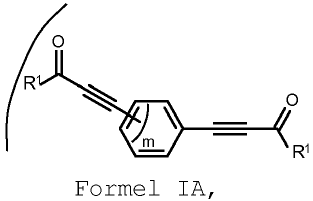 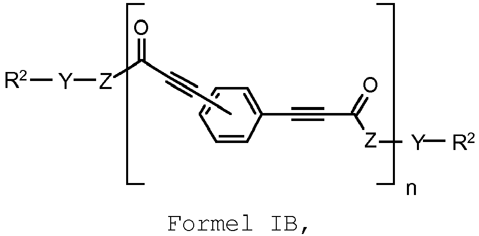 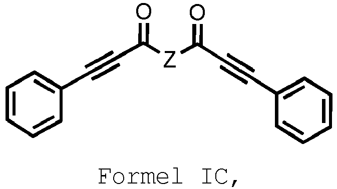 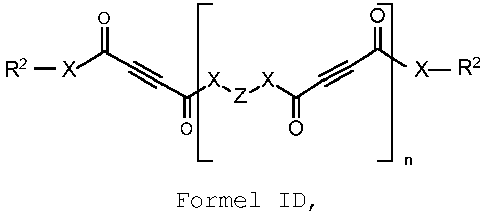 in denen
- R¹: jeweils unabhängig für einen aliphatischen linearen oder verzweigten C₁-C₂₀-Alkyl-Rest, der durch -O- oder -S- unterbrochen sein kann, oder für einen Phenylrest steht, der einen oder mehrere Substituenten tragen kann, die vorzugsweise unabhängig voneinander aus der Gruppe bestehend aus CH₃, C₂H₅, OH und OCH₃ ausgewählt sind,
- R²: jeweils unabhängig für H, einen aliphatischen linearen oder verzweigten C₁-C₂₀-Alkyl-Rest, der durch -O- oder -S- unterbrochen sein kann, oder für einen Phenylrest steht, der einen oder mehrere Substituenten tragen kann, die vorzugsweise unabhängig voneinander aus der Gruppe bestehend aus CH₃, C₂H₅, OH und OCH₃ ausgewählt sind,
- X: jeweils unabhängig für O, S, NH oder CH₂ steht,
- Y: jeweils unabhängig für -C(O)-O-, -O-C(O)-, O oder S steht,
- Z: für einen aliphatischen linearen oder verzweigten C₁-C₂₀-Alkylen-Rest, der durch -O- oder -S- unterbrochen sein kann, oder für einen Phenylenrest steht, der ein oder mehrere Substituenten tragen kann, die vorzugsweise unabhängig voneinander aus der Gruppe bestehend aus CH₃, C₂H₅, OH und OCH₃ ausgewählt sind,
- m: jeweils unabhängig die Werte 1 bis 5 annehmen kann und
- n: jeweils unabhängig die Werte 2 bis 10 und insbesondere die Werte 3 bis 8 annehmen kann.

Besonders bevorzugt sind solche Verbindungen der Formeln IA bis ID, bei denen jeweils unabhängig voneinander
- R¹: jeweils unabhängig für einen aliphatischen linearen oder verzweigten C₁-C₁₀-Alkyl-Rest, insbesondere C₁-C₆-Alkyl-Rest und vorzugsweise C₁-C₃-Alkyl-Rest steht, der durch -O- oder -S- unterbrochen sein kann,
- R²: jeweils unabhängig für H, einen aliphatischen linearen oder verzweigten C₁-C₁₀-Alkyl-Rest, insbesondere C₁-C₆-Alkyl-Rest und vorzugsweise C₁-C₃-Alkyl-Rest steht, der durch -O- oder -S- unterbrochen sein kann,
- X: für O, S, NH oder CH₂ steht,
- Y: jeweils unabhängig für -C(O)-O- oder -O-C(O)- steht,
- Z: für einen aliphatischen linearen oder verzweigten C₁-C₁₀-Alkylen-Rest, insbesondere C₁-C₆-Alkylen-Rest und vorzugsweise C₁-C₃-Alkylen-Rest, der durch -O- oder -S- unterbrochen sein kann, oder für einen Phenylenrest steht, der ein oder mehrere Substituenten tragen kann, die vorzugsweise unabhängig voneinander aus der Gruppe bestehend aus CH₃, C₂H₅, OH und OCH₃ ausgewählt sind,
- m: jeweils unabhängig die Werte 1, 2 oder 3 annehmen kann und
- n: jeweils unabhängig die Werte 3 bis 8 und insbesondere die Werte 4 bis 6 annehmen kann.

Besonders bevorzugt sind dabei Verbindungen, in denen alle Variablen eine der oben definierten Bedeutungen und insbesondere eine der bevorzugten Bedeutungen haben.

Die erfindungsgemäss verwendeten Dentalwerkstoffe enthalten üblicherweise ferner mindestens ein multifunktionelles Azid. Bevorzugt sind Dentalwerkstoffe, die mindestens ein Azid der Formel II enthalten

A-[X-Q-(Y-N₃)ₙ]ₘ Formel II,

in der
- A: für -O-, -N=, -NR³-, =N-N=, -NR³-N=, =N-NR³-, -NR³-NR³- oder einen m-wertigen Alkylen-Rest, Cycloalkylen-Rest, Heterocycloalkylen-Rest, Arylen-Rest, Heteroarylen-Rest, gemischten Alkylen-Cyclyl-Rest oder Cyclyl-O-Cyclyl-Rest steht, wobei Alkylen jeweils unabhängig für einen linearen oder verzweigten aliphatischen C₁-C₅₀-Rest steht, der durch ein oder mehrere -O-, -S-, -NR³-, -CO-O-, -O-CO-, -O-CO-O-, -CO-NR³-, -NR³-CO-, -O-CO-NR³-, -NR³-CO-O- oder -NR³-CO-NR³- unterbrochen sein kann, Cycloalkylen jeweils unabhängig für einen cycloaliphatischen C₃-C₁₈-Rest steht,
Heterocycloalkylen jeweils unabhängig für einen heterocycloaliphatischen C₃-C₁₈-Rest steht,
Arylen jeweils unabhängig für einen aromatischen C₆-C₁₈-Rest steht,
Heteroarylen jeweils unabhängig für einen heteroaromatischen C₃-C₁₈-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₅-Alkyl, OH, OCH₃ und OCOCH₃, tragen können,
- Q: jeweils unabhängig entfällt oder für einen (n+1)-wertigen linearen oder verzweigten aliphatischen C₁-C₂₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OH, OCH₃ und OCOCH₃, tragen können,
- R³: jeweils unabhängig für H oder einen C₁-C₁₀-Alkylrest steht,
- X und Y: jeweils unabhängig entfallen oder für -O-, -S-, -NR³-, -CO-, -CO-O-, -O-CO-, -O-CO-O-, -CO-NR³-, -NR³-CO-, -O-CO-NR³-, - NR³-CO-O- oder -NR³-CO-NR³- stehen,
- m: jeweils unabhängig die Werte 2 bis 6 annehmen kann und
- n: jeweils unabhängig die Werte 1 bis 4 annehmen kann.

Besonders bevorzugt sind Azide der Formel II, bei denen jeweils unabhängig voneinander
- A: für -N= oder einen m-wertigen Alkylen-Rest, Cycloalkylen-Rest, Heterocycloalkylen-Rest, Arylen-Rest, Heteroarylen-Rest, gemischten Alkylen-Cyclyl-Rest wie Alkylen-Cyclyl-Rest, Cyclyl-Alkylen-Rest, Cyclyl-Alkylen-Cyclyl-Rest oder Alkylen-Cyclyl-Alkylen-Cyclyl-Alkylen-Rest oder Cyclyl-O-Cyclyl-Rest steht, wobei Alkylen jeweils unabhängig für einen linearen oder verzweigten aliphatischen C₁-C₂₀-Rest steht, der durch ein oder mehrere -O-, -S-, -CO-O-, -O-CO- oder -O-CO-O- unterbrochen sein kann, Cycloalkylen jeweils unabhängig für einen cycloaliphatischen C₃-C₁₂-Rest steht,
Heterocycloalkylen jeweils unabhängig für einen heterocycloaliphatischen C₃-C₁₂-Rest steht,
Arylen jeweils unabhängig für einen aromatischen C₆-C₁₂-Rest steht,
Heteroarylen jeweils unabhängig für einen heteroaromatischen C₃-C₁₂-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₃-Alkyl, OCH₃ und OCOCH₃, tragen können,
- Q: jeweils unabhängig entfällt oder für einen (n+1)-wertigen linearen oder verzweigten aliphatischen C₁-C₁₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OCH₃ und OCOCH₃, tragen können,
- R³: jeweils unabhängig für H oder einen C₁-C₅-Alkylrest und insbesondere einen C₁-C₃-Alkylrest steht,
- X und Y: jeweils unabhängig entfallen oder für -O-, -S-, -CO-O-, -O-CO-, -O-CO-O-, -CO-NR³-, -NR³-CO-, -O-CO-NR³-, -NR³-CO-O- oder -NR³-CO-NR³- stehen,
- m: jeweils unabhängig die Werte 2, 3 oder 4 annehmen kann,
- n: jeweils unabhängig die Werte 1, 2 oder 3 und insbesondere 1 oder 2 annehmen kann und am meisten bevorzugt 1 ist.

Besonders bevorzugt sind Verbindungen, in denen alle Variablen eine der oben definierten Bedeutungen und insbesondere eine der bevorzugten Bedeutungen haben.

Ausserdem sind Dentalwerkstoffe bevorzugt, in denen Alkingruppen und Azidgruppen ganz oder annähernd im stöchiometrischen Verhältnis vorliegen und insbesondere im Verhältnis 2:1 - 1:2, vorzugsweise im Verhältnis 1,5:1 - 1:1,5, besonders bevorzugt im Verhältnis 1,1:1 - 1:1,1 und am meisten bevorzugt im Verhältnis 1,05:1 - 1:1,05 vorliegen.

Weiter bevorzugt sind solche Dentalwerkstoffe, in denen die Verbindungen der Formel I oder I', insbesondere der Formeln IA bis ID, sowie die multifunktionellen Azide eine mittlere Funktionalität von > 2,2 und insbesondere > 2,5 aufweisen. Unter der mittleren Funktionalität wird die durchschnittliche Anzahl an Alkin- bzw. Azidgruppen bezogen auf die in dem Dentalwerkstoff enthaltenen Moleküle von Verbindungen der Formel I oder I', insbesondere der Formeln IA bis ID, bzw. der multifunktionellen Azide verstanden.

Es wurde überraschend gefunden, dass die erfindungsgemäss verwendeten Dentalwerkstoffe, die mindestens ein Alkin mit der Formel I oder I', insbesondere einer der Formeln IA bis ID, und mindestens ein multifunktionelles Azid enthalten, hervorragend polymerisierbar sind und einen sehr hohen Umsatz der polyreaktionsfähigen Gruppen zeigen und nach der Aushärtung verbesserte mechanische Eigenschaften wie einen deutlich erhöhten Elastizitätsmodul aufweisen. Zudem können diese Dentalwerkstoffe ohne Metallkatalysatoren ausgehärtet werden und weisen bei der Härtungsreaktion eine relativ geringe exotherme Wärmetönung auf. Dadurch werden unkontrollierbare Reaktionsverläufe und insbesondere eine thermische Zersetzung der gebildeten Polyaddukte vermieden.

Darüber hinaus hat sich gezeigt, dass auf Basis der erfindungsgemäss verwendeten Dentalwerkstoffe durch geeignete Auswahl der multifunktionellen Azide der Formel I oder I', insbesondere der Formeln IA bis ID, und der multifunktionellen Azide, insbesondere Azide der Formel II, sowie durch Variation der mittleren Funktionalität dieser Verbindungen in dem Dentalwerkstoff durch Einstellung eines geeigneten Mischungsverhältnis insbesondere der Azide und Alkine, Polymernetzwerke mit massgeschneiderten Eigenschaften hergestellt werden können. Dabei nimmt die Netzwerkdichte mit der mittleren Funktionalität der Verbindungen und dem erreichten Umsatz der polymerisierbaren Gruppen zu. Durch Variation der Struktur der Alkine und/oder Azide, wie etwa die Verwendung von flexiblen oder steifen Spacern zur Anbindung der Azid- bzw. Alkin-Gruppen, lassen sich die Eigenschaften der gebildeten Polycycloadditionsnetzwerke weiter beeinflussen. Ausserdem können durch thermische Nachbehandlung der Umsetzungsgrad der funktionellen Gruppen und damit die Vernetzungsdichte zusätzlich erhöht werden. Schliesslich lassen sich die mechanischen Eigenschaften durch Zugabe von Füllstoffen weiter verbessern. Die erfindungsgemäss verwendeten Dentalwerkstoffe eignen sich daher insbesondere für die Herstellung von dentalen Restaurationsmaterialien wie hochmoduligen Komposit-Frässkörpern für CAD-CAM-basierte Verarbeitungstechniken zur Herstellung von zahnfarbenen Inlays, Onlays, Brücken oder Kronen.

Verbindungen der Formeln I und I' und insbesondere der Formeln IA bis ID lassen sich nach an sich bekannten Synthesemethoden einfach herstellen.

Die niedermolekularen oder oligomeren di- oder höherfunktionalisierten Alkine der Formeln IA bis ID lassen sich nach an sich bekannten Synthesemethoden einfach herstellen.

So sind niedermolekulare di- oder höherfunktionalisierte aktivierte Alkine der Formel IA ausgehend von di- oder höher alkinyliertem Benzol durch Ethinylierung mit geeigneten Carbonsäureestern zugänglich:

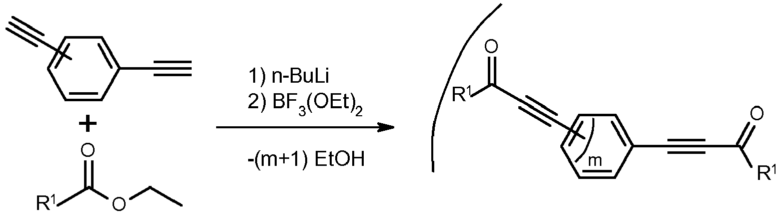

### Konkretes Beispiel:

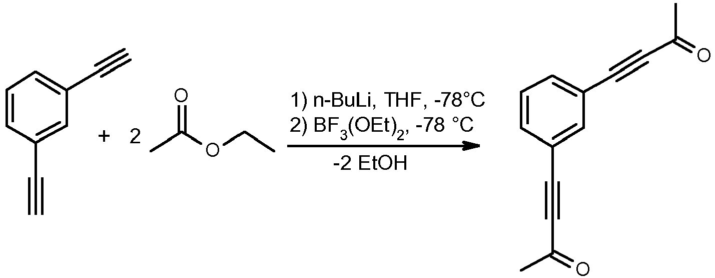

Analog dazu sind die oligomeren Alkine der Formel IB durch Ethinylierung mit geeigneten Dicarbonsäureestern zugänglich.

Oligomere höherfunktionalisierte aktivierte Alkine der Formel ID sind ausgehend von Acetylendicarboxylaten durch Umsetzung mit Verbindungen, die mit OH-Gruppen terminiert sind, zugänglich:

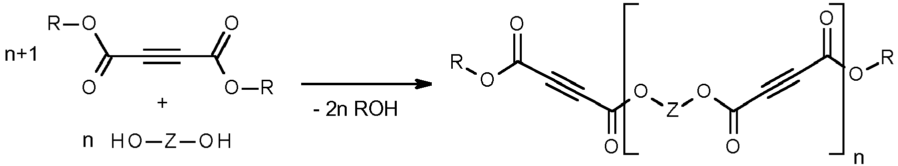

### Konkretes Beispiel:

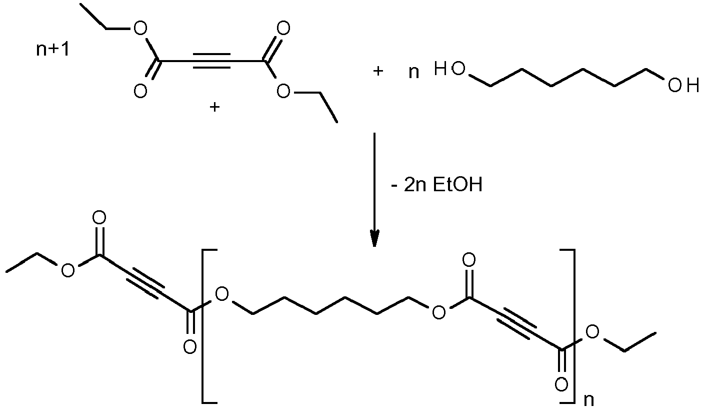

Beispiele für erfindungsgemäss verwendete Verbindungen der Formeln IA bis ID sind:

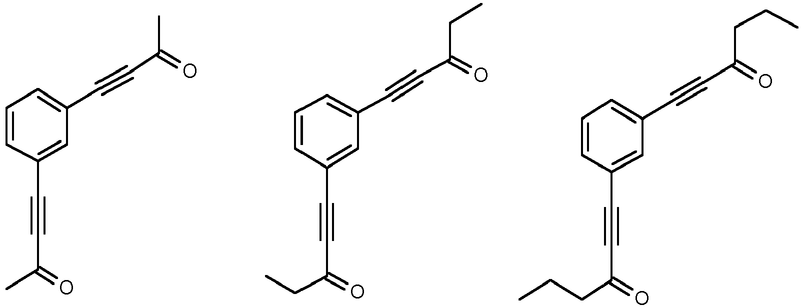

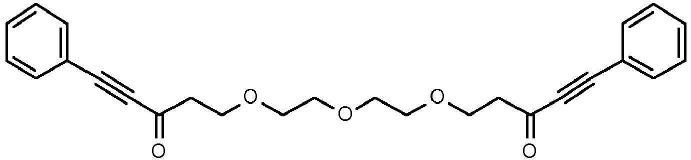

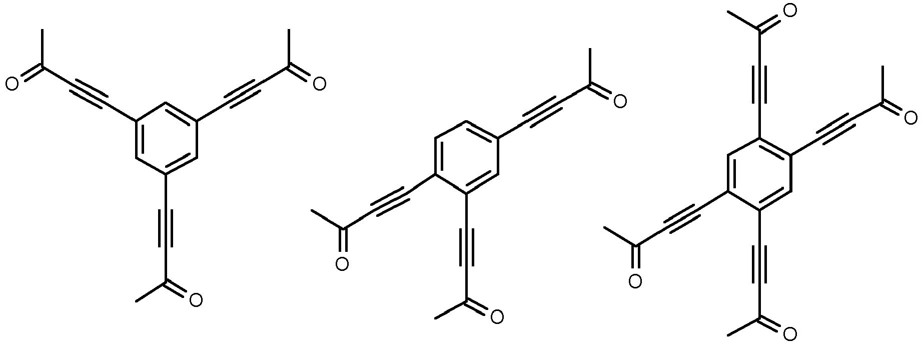

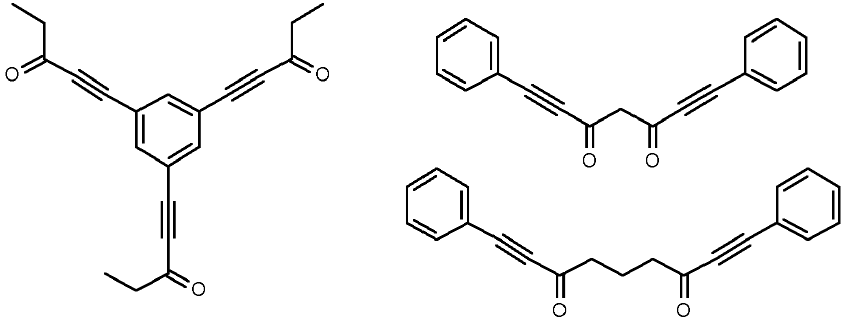

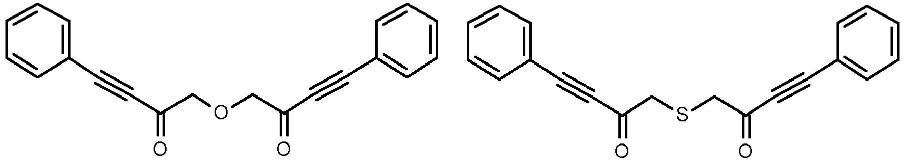

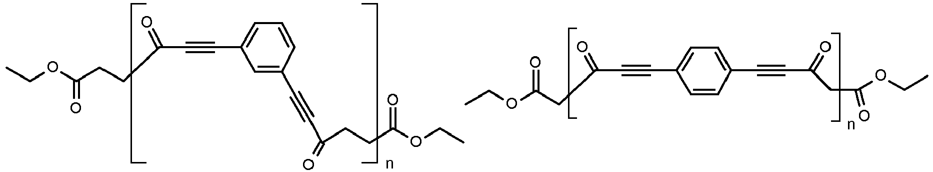

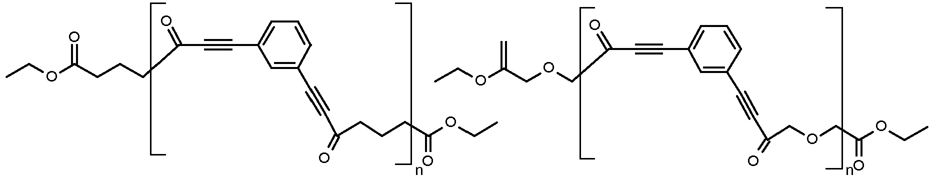

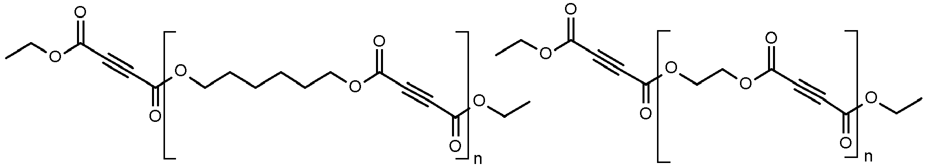

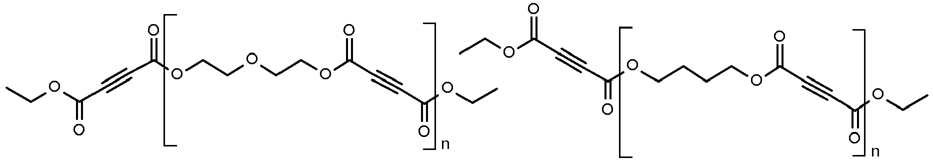

Auch Verbindungen der Formel II sind nach an sich bekannten Synthesemethoden leicht erhältlich. So sind Azide der Formel II beispielsweise durch nucleophile Substitution von geeigneten di- oder höherfunktionalisierten Halogeniden (Z=Cl,Br,I) oder p-Toluolsulfonsäureestern (Z=OTos) mit Natriumazid NaN₃ zugänglich:

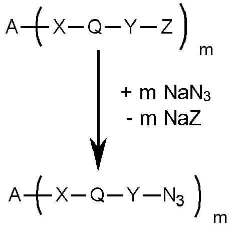

### Konkretes Beispiel: Umsetzung von 2,2,2-Tri(chlormethyl)ethanol mit Natriumazid

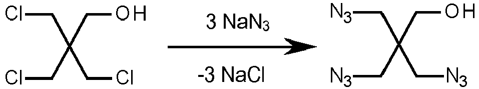

Weiterhin lassen sich Azidgruppen in an sich bekannter Weise aus aromatischen Hydrazinen durch Diazotierung, aus Alkoholen durch Mitsunobo-Reaktion oder aus Isocyanaten durch Addition von 2-Azidoethanol herstellen.

### Konkretes Beispiel: Umsetzung von Trimethylhexamethylendiisocyanat mit 2-Azidoethanol oder mit 2-Bromethanol gefolgt von Natriumazid

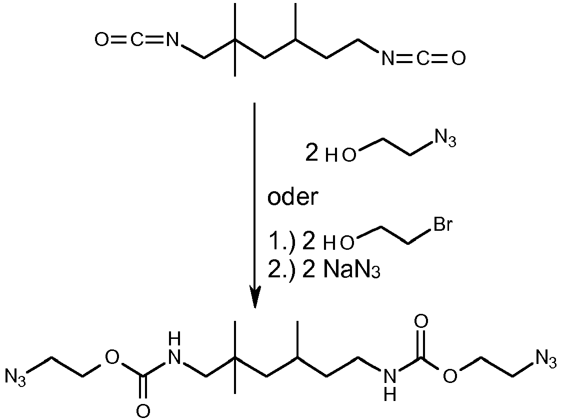

Beispiele für erfindungsgemäss verwendete Verbindungen der Formel II sind:

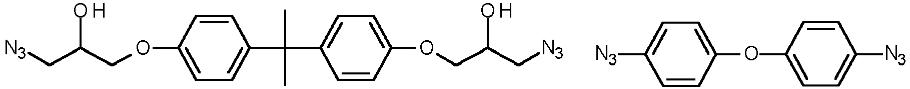

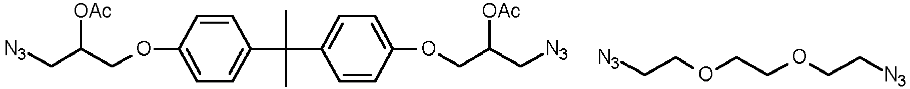

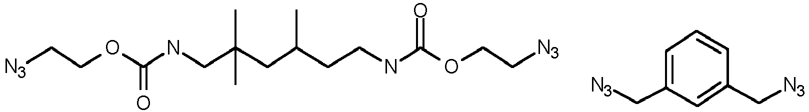

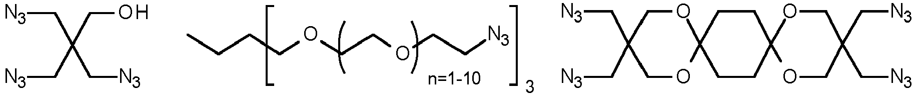

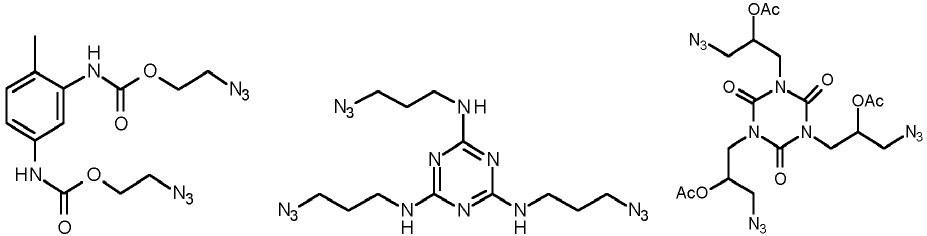

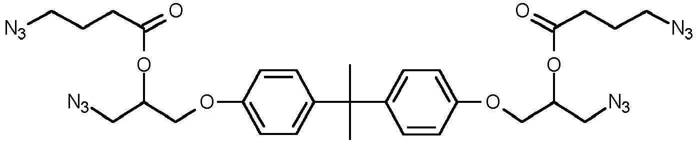

Die erfindungsgemäss verwendeten Dentalwerkstoffe enthalten vorzugsweise keinen Metallkatalysator.

Erfindungsgemäss können auch Hybridmaterialien eingesetzt werden, die neben mindestens einer Verbindung der Formel I oder I', insbesondere mindestens einer Verbindung mit einer der Formeln IA bis ID, und mindestens einem multifunktionellen Azid, insbesondere mindestens einem Azid der Formel II, radikalisch polymerisierbare Monomere enthalten. Solche Hybridmaterialien können stufenweise (erst thermisch und dann lichtinduziert) oder simultan (thermisch) ausgehärtet werden.

Als radikalisch polymerisierbare Monomere (Co-Monomere) sind insbesondere mono- oder polyfunktionelle (Meth)acrylsäurederivate geeignet. Unter monofunktionellen (Meth)acrylsäurederivaten werden Verbindungen mit einer, unter polyfunktionellen (Meth)acrylsäure-derivaten Verbindungen mit zwei oder mehr, vorzugsweise 2 bis 4 (Meth)acrylsäuregruppen verstanden. Polyfunktionelle Monomere haben eine vernetzende Wirkung.

Bevorzugte mono- oder polyfunktionelle (Meth)acrylsäurederivate sind Methyl-, Ethyl-, Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-GMA (ein Additionsprodukt aus Methacrylsäure und Bisphenol-A-diglycidylether), ethoxy- oder propoxyliertes Bisphenol-A-Dimethacrylat, wie etwa das Bisphenol-A-Dimethacrylat SR-348c (Sartomer) mit 3 Ethoxygruppen oder 2,2-Bis-[4-(2-methacryloxypropoxy)-phenyl]-propan, UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Glycerindi- und -tri(meth)acrylat, 1,4-Butandioldi(meth)acrylat, 1,10-Decandioldi(meth)acrylat (D₃MA) und 1,12-Dodecandioldi(meth)-acrylat.

Besonders bevorzugte mono- oder polyfunktionelle (Meth)acrylsäurederivate sind N-mono- oder -disubstitiuierte Acrylamide wie N-Ethylacrylamid, N,N-Dimethacrylamid, N-(2-Hydroxyethyl)acrylamid oder N-Methyl-N-(2-hydroxyethyl)acrylamid, N-monosubstituierte Methacrylamide wie N-Ethylmethacrylamid oder N-(2-Hydroxyethyl)-methacrylamid sowie N-Vinylpyrrolidon und Allylether. Diese Monomere zeichnen sich durch eine hohe Hydrolysestabilität aus und eignen sich aufgrund ihrer relativ geringen Viskosität besonders als Verdünnermonomere.

Bevorzugte polyfunktionelle (Meth)acrylsäurederivate mit hoher Hydrolysestabilität sind vernetzende Pyrrolidone wie 1,6-Bis-(3-vinyl-2-pyrrolidonyl)-hexan, Bisacrylamide wie Methylen- oder Ethylenbisacrylamid und Bis(meth)acrylamide wie N,N'-Diethyl-1,3-bis-(acrylamido)-propan, 1,3-Bis(methacrylamido)-propan, 1,4-Bis-(acrylamido)-butan oder 1,4-Bis(acryloyl)-piperazin, die durch Umsetzung aus den entsprechenden Diaminen mit (Meth)acrylsäurechlorid synthetisiert werden können.

Vorzugsweise werden dabei Mischungen der vorstehend genannten Monomere verwendet.

Hybridmaterialien enthalten ausserdem vorzugsweise einen Initiator für die radikalische Polymerisation.
Zur Initiierung der radikalischen Photopolymerisation werden vorzugsweise Benzophenon, Benzoin sowie deren Derivate oder α-Diketone oder deren Derivate wie 9,10-Phenanthrenchinon, 1-Phenyl-propan-1,2-dion, Diacetyl oder 4,4'-Dichlorbenzil eingesetzt. Besonders bevorzugt werden Campherchinon und 2,2-Dimethoxy-2-phenyl-acetophenon und ganz besonders bevorzugt α-Diketone in Kombination mit Aminen wie 4-(Dimethylamino)-benzoesäureester, N,N-Dimethylaminoethylmethacrylat, N,N-Dimethyl-sym.-xylidin oder Triethanolamin als Reduktionsmittel verwendet. Besonders geeignet sind auch Norrish-Typ-I-Photoinitiatoren, insbesondere Acyl- oder Bisacylphosphinoxide, Monoacyltrialkyl- oder Diacyldialkylgermanium-Verbindungen wie Benzoyltrimethylgermanium, Dibenzoyldiethylgermanium oder Bis-(4-methoxybenzoyl)diethylgermanium. Dabei lassen sich auch Mischungen der verschiedenen Photoinitiatoren wie beispielsweise Dibenzoyldiethylgermanium in Kombination mit Campherchinon und 4-Dimethylaminobenzoesäureethylester einsetzen.

Als Initiatoren für eine bei Raumtemperatur durchgeführte radikalische Polymerisation werden vorzugsweise Redox-Initiatorkombinationen wie beispielsweise Kombinationen von Benzoylperoxid mit N,N-Dimethyl-sym.-xylidin oder N,N-Dimethyl-p-toluidin verwendet. Darüber hinaus sind auch Redoxsysteme bestehend aus Peroxiden und solchen Reduktionsmitteln wie z.B. Ascorbinsäure, Barbituraten oder Sulfinsäuren besonders geeignet.

In einer bevorzugten Ausführungsform enthalten die erfindungsgemäss verwendeten Dentalwerkstoffe keine radikalisch polymerisierbaren Monomere und keine Initiatoren für die radikalische Polymerisation.

Weiterhin enthalten die erfindungsgemäss verwendeten Dentalwerkstoffe zur Verbesserung der mechanischen Eigenschaften oder zur Einstellung der Viskosität vorzugsweise auch organische oder anorganische Füllstoffpartikel.

Bevorzugte anorganische partikuläre Füllstoffe sind amorphe kugelförmige Materialien auf Basis von Oxiden wie ZrO₂ und TiO₂ oder Mischoxiden aus SiO₂, ZrO₂ und/oder TiO₂ mit einer mittleren durchschnittlichen Partikelgrösse von 0,005 bis 2 µm, vorzugsweise 0,1 bis 1 µm, nanopartikuläre oder mikrofeine Füllstoffe wie pyrogene Kieselsäure oder Fällungskieselsäure mit einer mittleren durchschnittlichen Partikelgrösse von 5 bis 200 nm, vorzugsweise 10 bis 100 nm, Minifüllstoffe wie Quarz-, Glaskeramik- oder Glaspulver beispielsweise von Barium- oder Strontiumaluminiumsilikatgläsern mit einer durchschnittlichen Teilchengrösse von 0,01 bis 15 µm, vorzugsweise 0,1 bis 1 µm, sowie röntgenopake Füllstoffe wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid mit einer mittleren durchschnittlichen Partikelgrösse von 10 bis 1000 nm, vorzugsweise 100 bis 300 nm. Die Bestimmung der durchschnittlichen Partikelgrösse kann insbesondere durch Transmissions- oder Rasterelektronenmikroskopie oder durch Laserbeugung erfolgen. Vorzugsweise kann im Bereich von 1 nm bis 50 nm die Transmissionselektronenmikroskopie, im Bereich von 50 nm bis 1 µm die Rasterelektronenmikroskopie und im Bereich von 1 µm bis 100 µm die Laserbeugung zur Bestimmung der durchschnittlichen Partikelgrösse verwendet werden. Geeignet sind auch faserförmige Füllstoffe, Nanofasern oder Wiskers.

Zur Verbesserung des Verbundes zwischen den Füllstoffpartikeln und der Polycycloadditionsmatrix können insbesondere Füllstoffe auf Basis von SiO₂ mit Silanen oberflächenmodifiziert werden, die Azid- oder Alkingruppen, insbesondere aktivierte Alkingruppen, tragen. Beispiele für solche Silane sind 3-Azidopropyltriethoxysilan und 4-[3-(Triethoxysilyl)propoxy]phenyl-but-3-in-2-on.

Ausserdem können die erfindungsgemäss verwendeten Dentalwerkstoffe weitere Additive enthalten, vor allem Stabilisatoren, Aromastoffe, Farbmittel, mikrobiozide Wirkstoffe, fluoridionenabgebende Additive, optische

Aufheller, Weichmacher, UV-Absorber oder Lösungsmittel wie Wasser oder Ethanol bzw. entsprechende Lösungsmittelgemische.

Besonders bevorzugt sind Dentalwerkstoffe auf Basis mindestens einer Verbindung der Formel I oder I', insbesondere mindestens einer Verbindung mit einer der Formeln IA bis ID, und mindestens einem multifunktionellen Azid, insbesondere mindestens einem Azid der Formel II, welche die folgenden Bestandteile enthalten:
a) 0,1 bis 70 Gew.-%, insbesondere 1 bis 60 Gew.-%, bevorzugt 5 bis 50 Gew.-% und besonders bevorzugt 10 bis 40 Gew.-% mindestens einer Verbindung der Formel I oder I', insbesondere mindestens einer Verbindung mit einer der Formeln IA bis ID,
b) 0,1 bis 70 Gew.-%, insbesondere 1 bis 60 Gew.-%, bevorzugt 5 bis 50 Gew.-% und besonders bevorzugt 10 bis 40 Gew.-% mindestens eines multifunktionellen Azids, insbesondere mindestens eines Azid der Formel II,
c) 0 bis 90 Gew.-%, bevorzugt 10 bis 80 Gew.-% und besonders bevorzugt 20 bis 70 Gew.-% Füllstoff und
d) 0 bis 70 Gew.-%, bevorzugt 1 bis 50 Gew.-% und besonders bevorzugt 5 bis 20 Gew.-% Lösungsmittel.

Die erfindungsgemäss verwendeten Dentalwerkstoffe eignen sich vor allem zur Herstellung von dentalen Kompositen, vorzugsweise Komposit-Formkörpern, die insbesondere für die maschinelle Bearbeitung mittels computergestützter Verarbeitungstechniken wie Fräs- und Schleifverfahren geeignet sind und sich vor allem zur Herstellung von dentalen Restaurationsmaterialien wie Inlays, Onlays, Kronen, Brücken oder Verblendmaterialien eignen.

Weiterhin betrifft die Erfindung auch die Verwendung mindestens einer Verbindung der Formel I oder I', insbesondere mindestens einer Verbindung mit einer der Formeln IA bis ID, und mindestens eines multifunktionellen Azids, insbesondere mindestens eines multifunktionellen Azids der Formel II, wie oben beschrieben zur Herstellung eines Dentalwerkstoffs und insbesondere eines dentalen Kompositmaterials.

Die Erfindung wird im Folgenden anhand von Ausführungsbeispielen näher erläutert.

### Ausführungsbeispiele

### Beispiel 1

### Synthese von 4,4'-(1,3-Phenylen)-bis-(but-3-in-2-on)

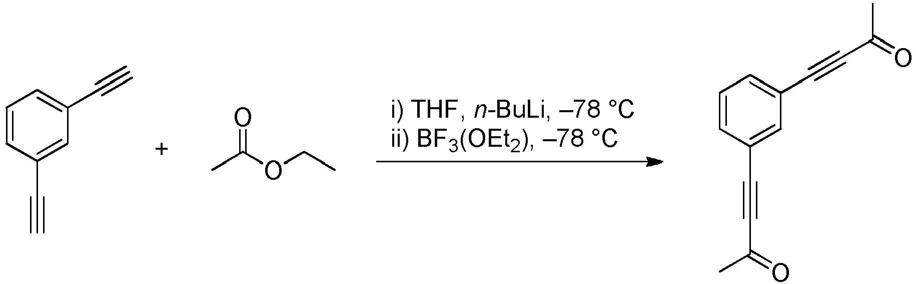

Die Synthese erfolgte analog zu M. Schuler et al., Angew. Chem. Int. Ed. 2008, 47, 7927-7930 (Supporting Information). 1,3-Diethinylbenzen (2,65 g, 21 mmol) wurde in getrocknetem THF (30 ml) gelöst und auf -78 °C abgekühlt. Danach wurde unter einer Stickstoffatmosphäre n-Butyllithium (1,6 M in n-Hexan, 30 ml, 48 mmol) zugetropft und 30 min gerührt. Anschliessend wurde eine Lösung aus Ethylacetat (4,8 ml, 48 mmol), THF (30 ml) und Bortrifluoriddiethyletherat (6.7 ml, 55 mmol) langsam zugegeben. Nach 30 min wurde die Reaktionsmischung mit einer gesättigten NH₄Cl-Lösung versetzt und auf Raumtemperatur gebracht. Die organische Phase wurde abgetrennt und die wässrige Phase noch zweimal mit Ethylacetat (50 ml) gewaschen. Nach dem Trocknen über wasserfreiem Na₂SO₄ wurde die Lösung eingeengt und das Produkt nach zweimaliger säulenchromatographischer Aufreinigung über Silica (1. CH₂Cl₂, 2. Hexan:Ethylacetat (1:1)) isoliert. Es wurden 240 mg (5% Ausbeute) des Produkts als orangefarbener Feststoff erhalten.
¹H-NMR (300 MHz, CDCl₃) : δ = 7,76 (m, 1H), 7,63 (dd, *J* = 7,8 Hz, *J* = 1,6 Hz, 2H), 7,43 (t, *J* = 7,8 Hz, 1H), 2,46 (s, 6H, CH₃). ¹³C-NMR (75 MHz, CDCl₃) : δ = 184,1, 136,9, 134,6, 129,1, 120,8, 88,7, 87,8, 32,7.

### Beispiel 2

### Synthese des Oligoesters 1

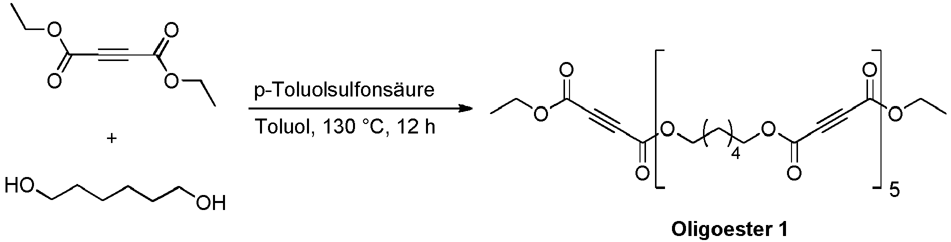

Diethylacetylendicarboxylat wurde gemäss M. S. Newman et al., J. Org. Chem. 1980, 45, 3523-3524 erhalten. In einem Einhalskolben wurden Diethylacetylendicarboxylat (4,675 g, 27,5 mmol), 1,6-Hexandiol (2,66 g, 22,5 mmol) und p-Toluolsulfonsäure (300 mg, 1,75 mmol) in Toluol (50 ml) gelöst und 12 h am Wasserabscheider bei 130 °C erhitzt. Die Reaktionslösung wurde dreimal mit wässriger NaHCO₃-Lösung gewaschen und anschliessend das Produkt mittels präparativer Grössenausschlusschromatographie (Bio-Beads S-X8, Eluent: CH₂Cl₂) abgetrennt. Es wurden 2,1 g (48% Ausbeute) des Produkts als gelbes Öl erhalten.
¹H-NMR (300 MHz, CDCl₃) : 4,27 (q, *J* = 7,1 Hz, 4H, C(O)O-CH₂-CH₃), 4,21 (t, *J* = 6,6 Hz, 4H, C(O)O-CH₂-(CH₂)4-CH₂-), 1,67 (m, 4H, C(O)O-CH₂-CH₂-(CH₂)₂-CH₂-), 1,39 (m, 4H, C(O)O-CH₂-CH₂-(CH₂)₂-), 1, 31 (t, *J* = 7,1 Hz, 6H, CH₃).
¹³C-NMR (75 MHz, CDCl₃) : 151, 8, 74, 7, 74, 6, 74, 5, 66, 7, 63, 0, 28, 0, 25,2, 13,8.
Mₙ (¹H-NMR) = 1150 g/mol (n = 5); durchschnittlicher Dreifachbindungsgehalt (¹H-NMR) = 6; Mₙ (SEC, PEG-Kalibration) = 1800 g/mol, M_{w} (SEC, PEG-Kalibration) = 8500 g/mol.

### Beispiel 3

### Synthese des Polymers 1 durch Azid-Alkin-Polycycloaddition mit einem aktivierten Alkin

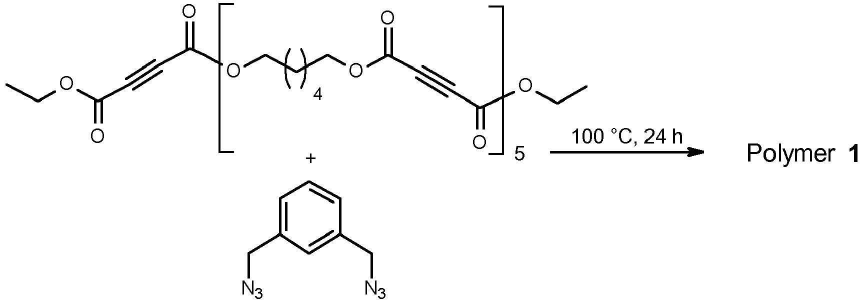

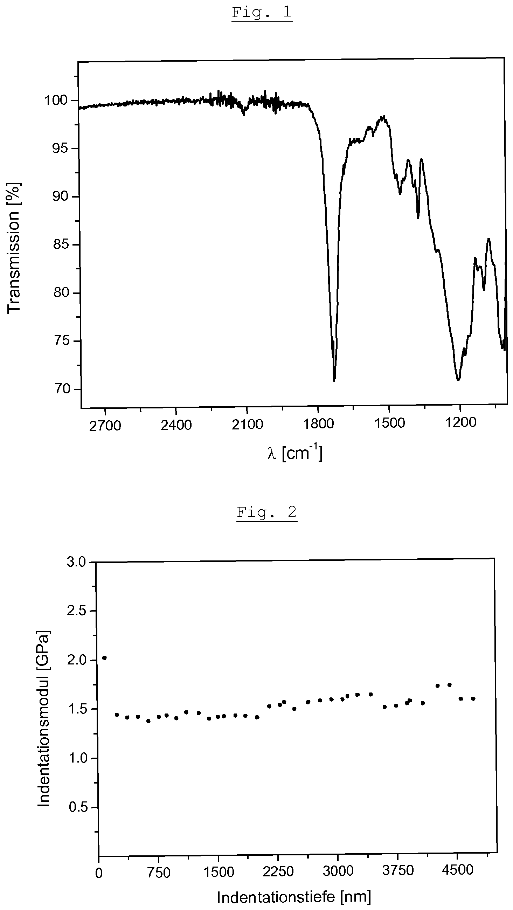

Oligoester 1 aus Beispiel 2 (200 mg) wurde in 1,3-Bis-(azidomethyl)-benzol (80 mg, 0,43 mmol, hergestellt nach S. T. Abu-Orabi, J. Chem. Eng. Data 1986, 31, 379-380) gelöst und die Lösung in eine Presshülse aus Teflon (Höhe 0,5-1,0 mm, Innendurchmesser 15 mm) gegeben. Anschliessend wurde bei 100 °C für 24 h auf einem Glasobjektträger auspolymerisiert. Man erhielt ein gelb-rotes Polymer **1.** Der Monomerumsatz wurde mittels ATR-IR-Spektroskopie durch Abnahme der IR-Absorption der Azidgruppe bei 2100 cm⁻¹ verfolgt. Das Spektrum nach 24 h ist in Fig. 1 dargestellt und zeigt einen Monomerumsatz von mehr als 95% an. Dann wurde der Elastizitätsmodul des Polyaddukts **1** mittels Nanoindentation bestimmt. Dabei handelt es sich um eine örtlich hochauflösende Methode zur mechanischen Charakterisierung von festen Stoffen, die auf der Messung von Kraft und Weg während des elastisch-plastischen Kontakts eines harten Prüfkörpers (Diamantindenter mit 4.7 µm Radius) mit der Probe basiert. Das Ergebnis ist in Fig. 2 gezeigt. Der Wert des mittels Nanoindentation bestimmten Elastizitätsmoduls betrug 1.5 ± 0.2 GPa.

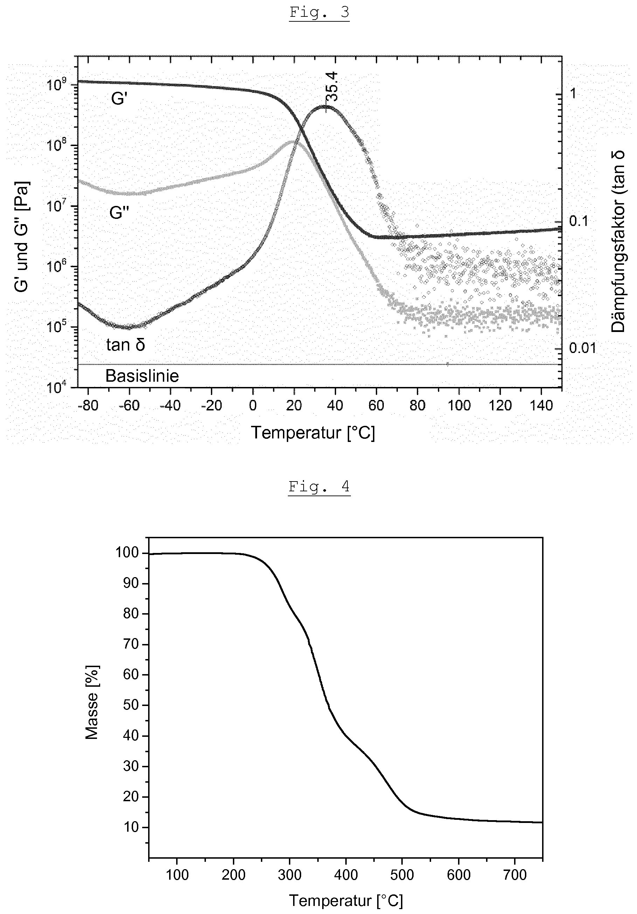

Zur Messung der Glasübergangstemperatur (Tg) wurde ein stabförmiger Prüfkörper des Polymers **1** angefertigt und anschliessend mittels dynamisch-mechanischer Thermoanalyse (DMTA-Messung am Rheometer Anton Paar MCR301) vermessen. Das Ergebnis der DMTA-Messung ist in Fig. 3 wiedergegeben. Die Glasübergangstemperatur, die sich aus dem lokalen Maximum der Verlustwinkel-Funktion (tan δ-Funktion) ergibt, betrug 35 °C.

Die thermische Stabilität von Polymer **1** wurde mittels thermogravimetrischen Analyse (TGA; Netzsch TG 209 F1 Iris) untersucht. Alle Messungen wurden unter einem Stickstoffstrom (20 ml/min) und in einem Temperaturbereich zwischen 20-800 °C (Heizrate: 20 K/min) durchgeführt. Das Ergebnis ist in Fig. 4 gezeigt. Polymer **1** zeigte sich bis zu einer Temperatur von ca. 260 °C stabil.

### Beispiel 4

### Wärmetönung von Azid-Alkin-Polycycloaddition mit nichtaktivierten und aktivierten Alkinen

Es wurden äquimolare binäre Mischungen der in der nachfolgenden Tabelle 1 aufgeführten Monomere hergestellt und deren Azid-Alkin-Polycycloaddition mittels dynamischer Differenzkalorimetrie (DSC) verfolgt. Die dynamische Differenzkalorimetrie (DSC) wurde an einem Netzsch DSC 204 F1 Phoenix-Instrument unter Stickstoffatmosphäre durchgeführt. Es wurden mindestens 10 mg Monomermischung in einem Aluminiumtiegel eingewogen und die Tiegel anschliessend verplombt. Alle Messungen wurden in einem Temperaturbereich von -20 bis 250 °C und mit einer Heizrate von 10 K min⁻¹ durchgeführt. Die so ermittelten Enthalpiewerte sind in Tabelle 1 zusammengestellt.

**Tabelle 1**

| **Polymer** | **Peak temperatur (°C)** | **Exotherme Reaktionsenthalpie** |
|---|---|---|
| | | ΔH' (kJ•mol⁻¹) |
| **Azid-Alkin Cycloadditionen mit nichtaktivierten Alkinen** | | |
| M1-M2 (kein Kupfer) | 136 | -560 |
| M1-M2 (1.0 mol% CuOAc) | 80 | -404 |
| M1-M2 (2.0 mol% CuOAc) | 65 | -400 |

| **Azid-Alkin Cycloaddition mit aktivierten Alkinen** | | |
|---|---|---|
| M1-**Oligoester 1** | 74 | -278 |
| M3-M4 **(Beispiel 1)** | 129 | -284 |

Der Vergleich der Ergebnisse zeigt, dass Mischungen mit aktivierten Alkinen sich durch eine deutlich geringere Wärmetönung auszeichnen.

### Beispiel 5

### Synthese eines Komposits durch Azid-Alkin-Polycycloaddition mit einem aktivierten Alkin

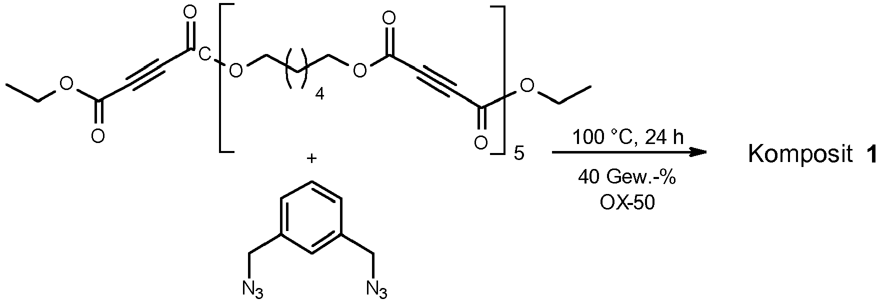

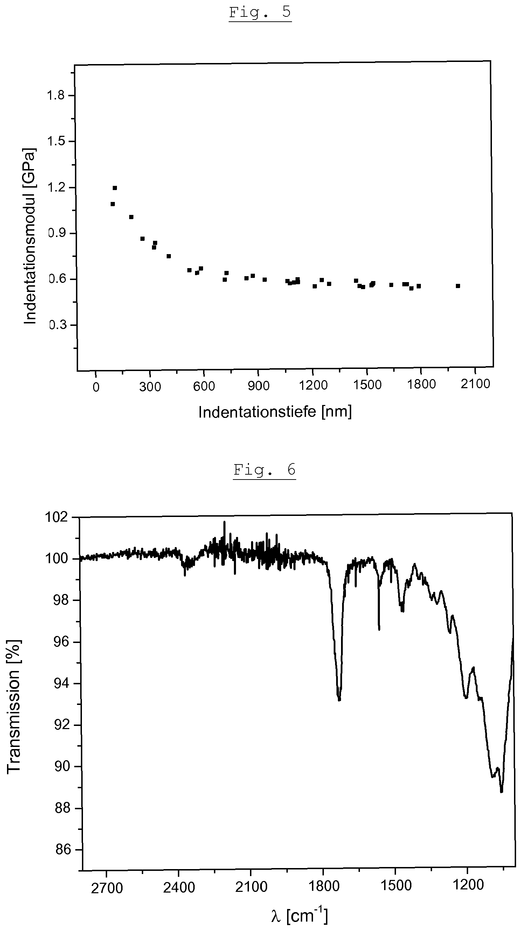

Oligoester 1 aus Beispiel 2 (200 mg) wurde in 1,3-Diazidomethylbenzen (80 mg, 0,43 mmol) gelöst. Anschliessend wurde der Füllstoff OX-50 (185 mg) zu der Lösung gegeben und so lange mit einem Spatel verrührt, bis eine homogene Masse entstanden war. Danach wurde die Reaktionsmischung bei 100 °C für 24 h auf einem Glasobjektträger auspolymerisiert. Man erhielt ein schwach gelbes Polymer mit einem Indentations-Modul von 2,4 ± 0,2 GPa (Fig. 5) und einem Monomerumsatz >90% (Fig. 6).

## Patentansprüche

1. Verwendung eines Dentalwerkstoffs, der mindestens eine Verbindung der Formel I oder I' enthält 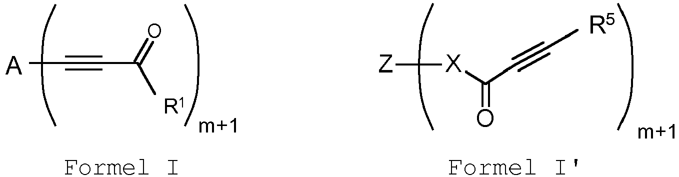 in denen
A jeweils unabhängig für einen (m+1)-wertigen Phenylenrest steht,
R¹ jeweils unabhängig für einen aliphatischen linearen oder verzweigten C₁-C₂₀-Alkyl-Rest, der durch -O- oder -S-unterbrochen sein kann, für einen Phenylrest, der einen oder mehrere Substituenten tragen kann, die vorzugsweise unabhängig voneinander aus der Gruppe bestehend aus CH₃, C₂H₅, OH und OCH₃ ausgewählt sind, oder für R³ steht,
R² jeweils unabhängig für H, einen aliphatischen linearen oder verzweigten C₁-C₂₀-Alkyl-Rest, der durch -O- oder -S-unterbrochen sein kann, oder für einen Phenylrest steht, der einen oder mehrere Substituenten tragen kann, die vorzugsweise unabhängig voneinander aus der Gruppe bestehend aus CH₃, C₂H₅, OH und OCH₃ ausgewählt sind,
R³ jeweils unabhängig für -Z-Y-R² oder -Z-C(=O)-≡-A(R⁴)ₘ steht,
R⁴ jeweils unabhängig für -≡-C(=O)-R³ steht,
R⁵ jeweils unabhängig für Phenyl oder R⁶ steht,
R⁶ jeweils unabhängig für -C(=O)-X-R² oder -C(=O)-X-Z(R⁷)m steht,
R⁷ jeweils unabhängig für -X-C(=O)-≡-R⁶ steht,
X jeweils unabhängig für O, S, NH oder CH₂ steht, wobei X entfällt, wenn R⁵ für Phenyl steht,
Y jeweils unabhängig für -C(O)-O-, -O-C(O)-, O oder S steht,
Z für einen (m+1)-wertigen aliphatischen linearen oder verzweigten C₁-C₂₀-Alkylen-Rest, der durch -O- oder -S-unterbrochen sein kann, oder für einen Phenylenrest steht, der ein oder mehrere Substituenten tragen kann, die vorzugsweise unabhängig voneinander aus der Gruppe bestehend aus CH₃, C₂H₅, OH und OCH₃ ausgewählt sind, und
m jeweils unabhängig die Werte 1 bis 5 annehmen kann,
zur Herstellung von dentalen Kompositen oder dentalen Restaurationsmaterialien.

2. Verwendung nach Anspruch 1, bei der in den Formeln I und I' jeweils unabhängig voneinander
A jeweils unabhängig für einen (m+1)-wertigen Phenylenrest steht,
R¹ jeweils unabhängig für einen aliphatischen linearen oder verzweigten C₁-C₁₀-Alkyl-Rest, insbesondere C₁-C₆-Alkyl-Rest und vorzugsweise C₁-C₃-Alkyl-Rest, der durch -O- oder -S- unterbrochen sein kann, oder für R³ steht,
R² jeweils unabhängig für H, einen aliphatischen linearen oder verzweigten C₁-C₁₀-Alkyl-Rest, insbesondere C₁-C₆-Alkyl-Rest und vorzugsweise C₁-C₃-Alkyl-Rest steht, der durch -O- oder -S- unterbrochen sein kann,
R³ jeweils unabhängig für -Z-Y-R² oder -Z-C(=O)-≡-A(R⁴)ₘ steht,
R⁴ jeweils unabhängig für -≡-C(=O)-R³ steht,
R⁵ jeweils unabhängig für Phenyl oder R⁶ steht,
R⁶ jeweils unabhängig für -C(=O)-X-R² oder -C(=O)-X-Z(R⁷)ₘ steht,
R⁷ jeweils unabhängig für -X-C(=O)-≡-R⁶ steht,
X jeweils unabhängig für O, S, NH oder CH₂ steht, wobei X entfällt, wenn R⁵ für Phenyl steht,
Y jeweils unabhängig für -C(O)-O- oder -O-C(O)- steht,
Z für einen (m+1)-wertigen aliphatischen linearen oder verzweigten C₁-C₁₀-Alkylen-Rest, insbesondere C₁-C₆-Alkylen-Rest und vorzugsweise C₁-C₃-Alkylen-Rest, der durch -O-oder -S- unterbrochen sein kann, oder für einen Phenylenrest steht, der ein oder mehrere Substituenten tragen kann, die vorzugsweise unabhängig voneinander aus der Gruppe bestehend aus CH₃, C₂H₅, OH und OCH₃ ausgewählt sind, und
m jeweils unabhängig die Werte 1, 2 oder 3 annehmen kann.

3. Verwendung nach Anspruch 1 oder 2, bei der der Dentalwerkstoff mindestens eine Verbindung mit einer der Formeln IA bis ID enthält 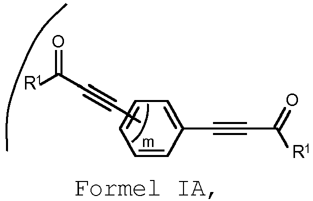 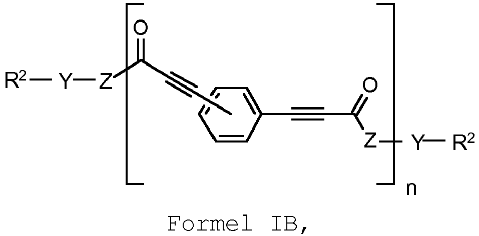 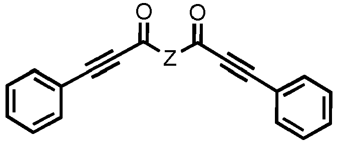 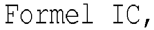 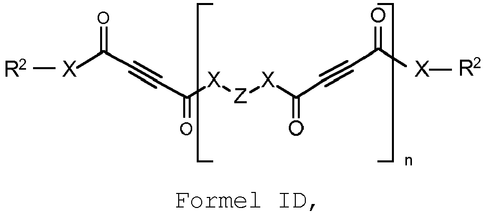 in denen
R¹ jeweils unabhängig für einen aliphatischen linearen oder verzweigten C₁-C₂₀-Alkyl-Rest, der durch -O- oder -S-unterbrochen sein kann, oder für einen Phenylrest steht, der einen oder mehrere Substituenten tragen kann, die vorzugsweise unabhängig voneinander aus der Gruppe bestehend aus CH₃, C₂H₅, OH und OCH₃ ausgewählt sind,
R² jeweils unabhängig für H, einen aliphatischen linearen oder verzweigten C₁-C₂₀-Alkyl-Rest, der durch -O- oder -S-unterbrochen sein kann, oder für einen Phenylrest steht, der einen oder mehrere Substituenten tragen kann, die vorzugsweise unabhängig voneinander aus der Gruppe bestehend aus CH₃, C₂H₅, OH und OCH₃ ausgewählt sind,
X jeweils unabhängig für O, S, NH oder CH₂ steht,
Y jeweils unabhängig für -C(O)-O-, -O-C(O)-, O oder S steht,
Z für einen aliphatischen linearen oder verzweigten C₁-C₂₀-Alkylen-Rest, der durch -O- oder -S- unterbrochen sein kann, oder für einen Phenylenrest steht, der ein oder mehrere Substituenten tragen kann, die vorzugsweise unabhängig voneinander aus der Gruppe bestehend aus CH₃, C₂H₅, OH und OCH₃ ausgewählt sind,
m jeweils unabhängig die Werte 1 bis 5 annehmen kann, und
n jeweils unabhängig die Werte 2 bis 10 annehmen kann,

4. Verwendung nach Anspruch 3, bei der in den Formeln IA bis ID jeweils unabhängig voneinander
R¹ jeweils unabhängig für einen aliphatischen linearen oder verzweigten C₁-C₁₀-Alkyl-Rest, insbesondere C₁-C₆-Alkyl-Rest und vorzugsweise C₁-C₃-Alkyl-Rest steht, der durch -O- oder -S- unterbrochen sein kann,
R² jeweils unabhängig für H, einen aliphatischen linearen oder verzweigten C₁-C₁₀-Alkyl-Rest, insbesondere C₁-C₆-Alkyl-Rest und vorzugsweise C₁-C₃-Alkyl-Rest steht, der durch -O- oder -S- unterbrochen sein kann,
X jeweils unabhängig für O, S, NH oder CH₂ steht,
Y jeweils unabhängig für -C(O)-O- oder -O-C(O)- steht,
Z für einen aliphatischen linearen oder verzweigten C₁-C₁₀-Alkylen-Rest, insbesondere C₁-C₆-Alkylen-Rest und vorzugsweise C₁-C₃-Alkylen-Rest, der durch -O- oder -S-unterbrochen sein kann, oder für einen Phenylenrest steht, der ein oder mehrere Substituenten tragen kann, die vorzugsweise unabhängig voneinander aus der Gruppe bestehend aus CH₃, C₂H₅, OH und OCH₃ ausgewählt sind,
m jeweils unabhängig die Werte 1, 2 oder 3 annehmen kann und
n jeweils unabhängig die Werte 3 bis 8 und insbesondere die Werte 4 bis 6 annehmen kann.

5. Verwendung nach einem der Ansprüche 1 bis 4, bei der der Dentalwerkstoff ferner mindestens ein Azid der Formel II enthält
A-[X-Q-(Y-N₃)ₙ]ₘ Formel II,
in der
A für -O-, -N=, -NR³-, =N-N=, -NR³-N=, =N-NR³-, -NR³-NR³- oder einen m-wertigen Alkylen-Rest, Cycloalkylen-Rest, Heterocycloalkylen-Rest, Arylen-Rest, Heteroarylen-Rest, gemischten Alkylen-Cyclyl-Rest oder Cyclyl-O-Cyclyl-Rest steht, wobei
Alkylen jeweils unabhängig für einen linearen oder verzweigten aliphatischen C₁-C₅₀-Rest steht, der durch ein oder mehrere -O-, -S-, -NR³-, -CO-O-, -O-CO-, -O-CO-O-, -CO-NR³-, -NR³-CO-, -O-CO-NR³-, -NR³-CO-O- oder -NR³-CO-NR³- unterbrochen sein kann,
Cycloalkylen jeweils unabhängig für einen cycloaliphatischen C₃-C₁₈-Rest steht,
Heterocycloalkylen jeweils unabhängig für einen heterocycloaliphatischen C₃-C₁₈-Rest steht,
Arylen jeweils unabhängig für einen aromatischen C₆-C₁₈-Rest steht,
Heteroarylen jeweils unabhängig für einen heteroaromatischen C₃-C₁₈-Rest steht,
Cyclyl jeweils unabhängig für Cycloalkylen, Heterocycloalkylen, Arylen oder Heteroarylen steht und die genannten Reste jeweils unabhängig einen oder mehrere Substituenten, insbesondere ausgewählt aus C₁-C₅-Alkyl, OH, OCH₃ und OCOCH₃, tragen können,
Q jeweils unabhängig entfällt oder für einen (n+1)-wertigen linearen oder verzweigten aliphatischen C₁-C₂₀-Alkylen-Rest, der durch ein oder mehrere -O- oder -S- unterbrochen sein kann, oder einen Phenylenrest steht, wobei die genannten Reste jeweils unabhängig einen oder mehrere Substituenten, insbesondere ausgewählt aus CH₃, C₂H₅, OH, OCH₃ und OCOCH₃, tragen können,
R³ jeweils unabhängig für H oder einen C₁-C₁₀-Alkylrest steht,
X und Y jeweils unabhängig entfallen oder für -O-, -S-, -NR³-, -CO-, -CO-O-, -O-CO-, -O-CO-O-, -CO-NR³-, -NR³-CO-, -O-CO-NR³-, -NR³-CO-O- oder -NR³-CO-NR³- stehen,
m jeweils unabhängig die Werte 2 bis 6 annehmen kann und
n jeweils unabhängig die Werte 1 bis 4 annehmen kann.

6. Verwendung nach Anspruch 5, bei der in Formel II jeweils unabhängig voneinander
A für -N= oder einen m-wertigen Alkylen-Rest, Cycloalkylen-Rest, Heterocycloalkylen-Rest, Arylen-Rest, Heteroarylen-Rest, gemischten Alkylen-Cyclyl-Rest oder Cyclyl-O-Cyclyl-Rest steht, wobei
Alkylen jeweils unabhängig für einen linearen oder verzweigten aliphatischen C₁-C₂₀-Rest steht, der durch ein oder mehrere -O-, -S-, -CO-O-, -O-CO- oder -O-CO-O- unterbrochen sein kann,
Cycloalkylen jeweils unabhängig für einen cycloaliphatischen C₃-C₁₂-Rest steht,
Heterocycloalkylen jeweils unabhängig für einen heterocycloaliphatischen C₃-C₁₂-Rest steht,
Arylen jeweils unabhängig für einen aromatischen C₆-C₁₂-Rest steht,
Heteroarylen jeweils unabhängig für einen heteroaromatischen C₃-C₁₂-Rest steht,
Cyclyl jeweils unabhängig für Cycloalkylen, Heterocycloalkylen, Arylen oder Heteroarylen steht und
die genannten Reste jeweils unabhängig einen oder mehrere Substituenten, insbesondere ausgewählt aus C₁-C₃-Alkyl, OCH₃ und OCOCH₃, tragen können,
Q jeweils unabhängig entfällt oder für einen (n+1)-wertigen linearen oder verzweigten aliphatischen C₁-C₁₀-Alkylen-Rest, der durch ein oder mehrere -O- oder -S-unterbrochen sein kann, oder einen Phenylenrest steht, wobei die genannten Reste jeweils unabhängig einen oder mehrere Substituenten, insbesondere ausgewählt aus CH₃, C₂H₅, OCH₃ und OCOCH₃, tragen können,
R³ jeweils unabhängig für H oder einen C₁-C₅-Alkylrest und insbesondere einen C₁-C₃-Alkylrest steht,
X und Y jeweils unabhängig entfallen oder für -O-, -S-, -CO-O-, -O-CO-, -O-CO-O-, -CO-NR³-, -NR³-CO-, -O-CO-NR³-, -NR³-CO-O- oder -NR³-CO-NR³- stehen,
m jeweils unabhängig die Werte 2, 3 oder 4 annehmen kann,
n jeweils unabhängig die Werte 1, 2 oder 3 und insbesondere 1 oder 2 annehmen kann und am meisten bevorzugt 1 ist.

7. Verwendung nach einem der Ansprüche 1 bis 6, bei der der Dentalwerkstoff ein oder mehrere radikalisch polymerisierbare Monomere enthält.

8. Verwendung nach Anspruch 7, bei der der Dentalwerkstoff
Methyl-, Ethyl-, Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-GMA, ethoxy- oder propoxyliertes Bisphenol-A-Dimethacrylat, UDMA, Di-, Tri- oder Tetraethylenglycoldi(meth)acrylat, Trimethylolpropantri(meth)acrylat, Pentaerythrittetra(meth)acrylat, Glycerindi- oder -tri(meth)acrylat, 1,4-Butandioldi(meth)acrylat, 1,10-Decandioldi(meth)acrylat (D₃MA), 1,12-Dodecandioldi(meth)-acrylat,
und/oder
ein oder mehrere N-mono- oder -disubstitiuierte Acrylamide, N-Ethylacrylamid, N,N-Dimethacrylamid, N-(2-Hydroxyethyl)-acrylamid, N-Methyl-N-(2-hydroxyethyl)acrylamid, ein oder mehrere N-monosubstituierte Methacrylamide, N-Ethylmethacrylamid, N-(2-Hydroxyethyl)methacrylamid, N-Vinylpyrrolidon, ein oder mehrere vernetzende Allylether,
und/oder
ein oder mehrere vernetzende Pyrrolidone, 1,6-Bis(3-vinyl-2-pyrrolidonyl)-hexan, ein oder mehrere vernetzende Bisacrylamide, Methylen- oder Ethylenbisacrylamid, ein oder mehrere vernetzende Bis(meth)acrylamide, N,N'-Diethyl-1,3-bis(acryl-amido)-propan, 1,3-Bis(methacrylamido)-propan, 1,4-Bis(acryl-amido)-butan, 1,4-Bis(acryloyl)-piperazin,
oder eine Mischung davon enthält.

9. Verwendung nach einem der Ansprüche 1 bis 8, bei der der Dentalwerkstoff organischen und/oder anorganischen Füllstoff enthält.

10. Verwendung nach Anspruch 9, bei der der Dentalwerkstoff mindestens einen anorganischen partikulären Füllstoff ausgewählt aus der Gruppe bestehend aus amorphen kugelförmigen Materialien auf Basis von Oxiden oder Mischoxiden mit einer mittleren durchschnittlichen Partikelgrösse von 0,005 bis 2 µm, vorzugsweise 0,1 bis 1 µm, nanopartikulären oder mikrofeinen Füllstoffen mit einer mittleren durchschnittlichen Partikelgrösse von 5 bis 200 nm, vorzugsweise 10 bis 100 nm, Minifüllstoffen mit einer durchschnittlichen Teilchengrösse von 0,01 bis 15 µm, vorzugsweise 0,1 bis 1 µm, sowie röntgenopaken Füllstoffen mit einer mittleren durchschnittlichen Partikelgrösse von 10 bis 1000 nm, vorzugsweise 100 bis 300 nm, enthält.

11. Verwendung nach einem der Ansprüche 1 bis 10, bei der der Dentalwerkstoff
a) 0,1 bis 70 Gew.-%, insbesondere 1 bis 60 Gew.-%, bevorzugt 5 bis 50 Gew.-% und besonders bevorzugt 10 bis 40 Gew.-% mindestens einer Verbindung der Formel I oder I', insbesondere mindestens einer Verbindung mit einer der Formeln IA bis ID,
b) 0,1 bis 70 Gew.-%, insbesondere 1 bis 60 Gew.-%, bevorzugt 5 bis 50 Gew.-% und besonders bevorzugt 10 bis 40 Gew.-% mindestens eines multifunktionellen Azids, insbesondere mindestens eines Azid der Formel II,
c) 0 bis 90 Gew.-%, bevorzugt 10 bis 80 Gew.-% und besonders bevorzugt 20 bis 70 Gew.-% Füllstoff und
d) 0 bis 70 Gew.-%, bevorzugt 1 bis 50 Gew.-% und besonders bevorzugt 5 bis 20 Gew.-% Lösungsmittel enthält.

12. Verwendung nach einem der Ansprüche 1 bis 11 zur Herstellung von Inlays, Onlays, Kronen, Brücken oder Verblendmaterialien.

13. Verwendung mindestens einer Verbindung der Formel I oder I' wie in Anspruch 1 oder 2 definiert, vorzugsweise mindestens einer Verbindung mit einer der Formeln IA bis ID wie in Anspruch 3 oder 4 definiert, und insbesondere mindestens eines Azids der Formel II wie in Anspruch 5 oder 6 definiert, zur Herstellung eines Dentalwerkstoffs und insbesondere eines dentalen Kompositmaterials.

## Claims

1. Use of a dental material that comprises at least one compound of Formula I or I' 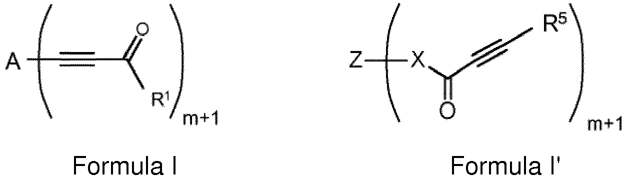 in which
A represents in each case independently an (m+1)-valent phenylene group,
R¹ represents in each case independently an aliphatic linear or branched C₁-C₂₀ alkyl group that can be interrupted by -O- or-S-, a phenyl group that can carry one or more substituents that are preferably selected independently of each other from the group consisting of CH₃, C₂H₅, OH and OCH₃, or R³,
R² represents in each case independently H, an aliphatic linear or branched C₁-C₂₀ alkyl group that can be interrupted by -O- or -S-, or a phenyl group that can carry one or more substituents that are preferably selected independently of each other from the group consisting of CH₃, C₂H₅, OH and OCH₃,
R³ represents in each case independently -Z-Y-R² or -Z-C(=O)-≡-A(R⁴)ₘ,
R⁴ represents in each case independently -≡-C(=O)-R³,
R⁵ represents in each case independently phenyl or R⁶,
R⁶ represents in each case independently -C(=O)-X-R² or -C(=O)-X-Z(R⁷)m,
R⁷ represents in each case independently -X-C(=O)-≡-R⁶,
X represents in each case independently O, S, NH or CH₂, wherein X is missing when R⁵ represents phenyl,
Y represents in each case independently -C(O)-O-, -O-C(O)-, O or S,
Z represents an (m+1)-valent aliphatic linear or branched C₁-C₂₀ alkylene group that can be interrupted by -O- or -S-, or a phenylene group that can carry one or more substituents that are preferably selected independently of each other from the group consisting of CH₃, C₂H₅, OH and OCH₃, and
m in each case independently can assume the values 1 to 5,
for manufacturing dental composites or dental restoration materials.

2. Use according to claim 1, **characterised in that** in the Formulae I and I' in each case independently of each other
A represents in each case independently an (m+1)-valent phenylene group,
R¹ represents in each case independently an aliphatic linear or branched C₁-C₁₀ alkyl group, in particular a C₁-C₆ alkyl group and preferably a C₁-C₃ alkyl group, which can be interrupted by -O- or - S-, or represents R³,
R² represents in each case independently H, an aliphatic linear or branched C₁-C₁₀ alkyl group, in particular a C₁-C₆ alkyl group and preferably a C₁-C₃ alkyl group, which can be interrupted by -O- or - S-,
R³ represents in each case independently -Z-Y-R² or -Z-C(=O)-≡-A(R⁴)ₘ,
R⁴ represents in each case independently -≡-C(=O)-R ³,
R⁵ represents in each case independently phenyl or R⁶,
R⁶ represents in each case independently -C(=O)-X-R² or -C(=O)-X-Z(R⁷)ₘ,
R⁷ represents in each case independently -X-C(=O)-≡-R⁶,
X represents in each case independently O, S, NH or CH₂, wherein X is missing when R⁵ represents phenyl,
Y represents in each case independently -C(O)-O- or -O-C(O)-,
Z represents an (m+1)-valent aliphatic linear or branched C₁-C₁₀ alkylene group, in particular a C₁-C₆ alkylene group and preferably a C₁-C₃ alkylene group, which can be interrupted by -O- or -S-, or represents a phenylene group that can carry one or more substituents that are preferably selected independently of each other from the group consisting of CH₃, C₂H₅, OH and OCH₃, and
m in each case independently can assume the values 1, 2 or 3.

3. Use according to claim 1 or 2, **characterised in that** the dental material comprises at least one compound having one of the Formulae IA to ID 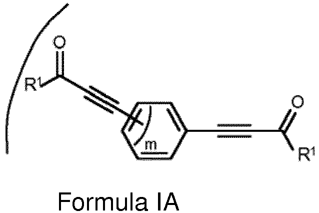 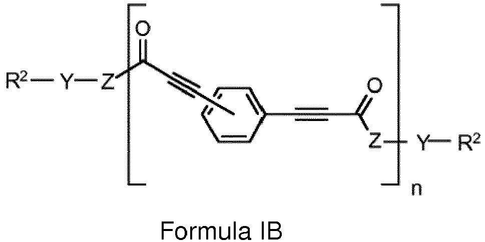 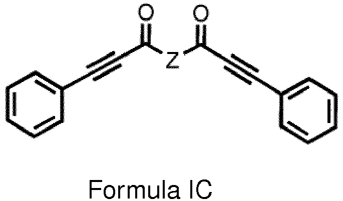 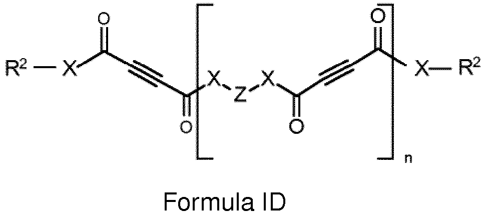 In which
R¹ represents in each case independently an aliphatic linear or branched C₁-C₂₀ alkyl group that can be interrupted by -O- or -S-, or a phenyl group that can carry one or more substituents that are preferably selected independently of each other from the group consisting of CH₃, C₂H₅, OH and OCH₃,
R² represents in each case independently H, an aliphatic linear or branched C₁-C₂₀ alkyl group that can be interrupted by -O- or -S-, or a phenyl group that can carry one or more substituents that are preferably selected independently of each other from the group consisting of CH₃, C₂H₅, OH and OCH₃,
X represents in each case independently O, S, NH or CH₂,
Y represents in each case independently -C(O)-O-, -O-C(O)-, O or S,
Z represents an aliphatic linear or branched C₁-C₂₀ alkylene group that can be interrupted by -O- or -S-, or a phenylene group that can carry one or more substituents that are preferably selected independently of each other from the group consisting of CH₃, C₂H₅, OH and OCH₃,
m in each case independently can assume the values 1 to 5, and
n in each case independently can assume the values 2 to 10.

4. Use according to claim 3, **characterised in that** in the Formulae IA to ID in each case independently of each other
R¹ represents in each case independently an aliphatic linear or branched C₁-C₁₀ alkyl group, in particular a C₁-C₆ alkyl group and preferably a C₁-C₃ alkyl group, which can be interrupted by -O- or - S-,
R² represents in each case independently H, an aliphatic linear or branched C₁-C₁₀ alkyl group, in particular a C₁-C₆ alkyl group and preferably a C₁-C₃ alkyl group, which can be interrupted by -O- or - S-,
X represents in each case independently O, S, NH or CH₂,
Y represents in each case independently -C(O)-O- or -O-C(O)-,
Z represents an aliphatic linear or branched C₁-C₁₀ alkylene group, in particular a C₁-C₆ alkylene group and preferably a C₁-C₃ alkylene group, which can be interrupted by -O- or -S-, or a phenylene group that can carry one or more substituents that are preferably selected independently of each other from the group consisting of CH₃, C₂H₅, OH and OCH₃,
m in each case independently can assume the values 1, 2 or 3, and
n in each case independently can assume the values 3 to 8 and in particular the values 4 to 6.

5. Use according to one of claims 1 to 4, **characterised in that** the dental material further comprises at least one azide of Formula II
A-[X-Q-(Y-N₃)ₙ]ₘ Formula II
In which
A represents -O-, -N=, -NR³-, =N-N=, -NR³-N=, =N-NR³-, -NR³-NR³-or an m-valent alkylene group, cycloalkylene group, heterocycloalkylene group, arylene group, heteroarylene group, mixed alkylene-cyclyl group or cyclyl-O-cyclyl group, wherein alkylene represents in each case independently a linear or branched aliphatic C₁-C₅₀ group that can be interrupted by one or more -O-, -S-, -NR³-, -CO-O-, -O-CO-, -O-CO-O-, -CO-NR³-, -NR³-CO-, -O-CO-NR³-, -NR³-CO-O- or -NR³-CO-NR³-,
cycloalkylene represents in each case independently a cycloaliphatic C₃-C₁₈ group,
heterocycloalkylene represents in each case independently a heterocycloaliphatic C₃-C₁₈ group,
arylene represents in each case independently an aromatic C₆-C₁₈ group,
heteroarylene represents in each case independently a heteroaromatic C₃-C₁₈ group,
cyclyl represents in each case independently cycloalkylene, heterocycloalkylene, arylene or heteroarylene and
the cited groups in each case independently can carry one or more substituents, in particular selected from C₁-C₅ alkyl, OH, OCH₃ and OCOCH₃,
Q in each case independently is missing or represents an (n+1)-valent linear or branched aliphatic C₁-C₂₀ alkylene group that can be interrupted by one or more -O- or -S-, or a phenylene group, wherein the cited groups in each case independently can carry one or more substituents, in particular selected from CH₃, C₂H₅, OCH₃ and OCOCH₃,
R³ in each case independently represents H or a C₁-C₁₀ alkyl group,
X and Y in each case independently are missing or represent -O-, -S-, - NR³-, -CO-, -CO-O-, -O-CO-, -O-CO-O-, -CO-NR³-, -NR³-CO-, -O-CO-NR³-, -NR³-CO-O- or -NR³-CO-NR³-,
m in each case independently can assume the values 2 to 6 and
n in each case independently can assume the values 1 to 4.

6. Use according to claim 5, **characterised in that** in the Formula II in each case independently of each other
A represents -N= or an m-valent alkylene group, cycloalkylene group, heterocycloalkylene group, arylene group, heteroarylene group, mixed alkylene-cyclyl group or cyclyl-O-cyclyl group, wherein
alkylene represents in each case independently a linear or branched aliphatic C₁-C₂₀ group that can be interrupted by one or more -O-, -S-, -CO-O-, -O-CO- or -O-CO-O-,
cycloalkylene represents in each case independently a cycloaliphatic C₃-C₁₂ group,
heterocycloalkylene represents in each case independently a heterocycloaliphatic C₃-C₁₂ group,
arylene represents in each case independently an aromatic C₆-C₁₂ group,
heteroarylene represents in each case independently a heteroaromatic C₃-C₁₂ group,
cyclyl represents in each case independently cycloalkylene, heterocycloalkylene, arylene or heteroarylene and the cited groups can in each case independently carry one or more substituents, in particular selected from C₁-C₃ alkyl, OCH₃ and OCOCH₃,
Q in each case independently is missing or represents an (n+1)-valent linear or branched aliphatic C₁-C₁₀ alkylene group that can be interrupted by one or more -O- or -S-, or a phenylene group, wherein the indicated groups in each case independently can carry one or more substituents, in particular selected from CH₃, C₂H₅, OCH₃ and OCOCH₃,
R³ in each case independently represents H or a C₁-C₅ alkyl group and in particular a C₁-C₃ alkyl group,
X and Y in each case independently are missing or represent -O-, -S-, - CO-O-, -O-CO-, -O-CO-O-, -CO-NR³-, -NR³-CO-, -O-CO-NR³-, - NR³-CO-O- or -NR³-CO-NR³-,
m in each case independently can assume the values 2, 3 or 4,
n in each case independently can assume the values 1, 2 or 3 and in particular 1 or 2, and most preferably is 1.

7. Use according to one of claims 1 to 6, **characterised in that** the dental material comprises one or more radically polymerisable monomers.

8. Use according to claim 7, **characterised in that** the dental material comprises
methyl, ethyl, hydroxyethyl, butyl, benzyl, tetrahydrofurfuryl or isobornyl (meth)acrylate, p-cumyl-phenoxyethylene glycol methacrylate (CMP-1E), bisphenol-A-di(meth)acrylate, Bis-GMA, ethoxylated or propoxylated bisphenol-A-dimethacrylate, UDMA, di-, tri- or tetraethylene glycol di(meth)acrylate, trimethylolpropane tri(meth)acrylate, pentaerythritol tetra(meth)acrylate, glycerol di- or tri(meth)acrylate, 1,4-butanediol di(meth)acrylate, 1,10-decanediol di(meth)acrylate (D₃MA), 1,12-dodecanediol di(meth)acrylate,
and/or
one or more *N*-mono or *N*-disubstituted acrylamides, *N*-ethylacrylamide, *N,N*-dimethacrylamide, *N*-(2-hydroxyethyl)acrylamide, *N*-methyl-*N*-(2-hydroxyethyl)acrylamide, one or more *N*-monosubstituted methacrylamides, *N*-ethylmethacrylamide, *N*-(2-hydroxyethyl)methacrylamide, *N*-vinyl pyrrolidone, one or more cross-linking allyl ethers,
and/or
one or more cross-linking pyrrolidones, 1,6-bis(3-vinyl-2-pyrrolidonyl)-hexane, one or more cross-linking bisacrylamides, methylene or ethylene bisacrylamide, one or more cross-linking bis(meth)acrylamides, *N,N*'-diethyl-1,3-bis(acrylamido)-propane, 1,3-bis(methacrylamido)-propane, 1,4-bis(acrylamido)-butane, 1,4-bis(acryloyl)-piperazine,
or a mixture thereof.

9. Use according to one of claims 1 to 8, **characterised in that** the dental material comprises organic and/or inorganic filler.

10. Use according to claim 9, **characterised in that** the dental material comprises at least one inorganic particulate filler selected from the group consisting of amorphous spherical materials based on oxides or mixed oxides having a mean particle size of from 0.005 to 2 µm, preferably 0.1 to 1 µm, nanoparticulate or microfine fillers having a mean particle size of from 5 to 200 nm, preferably 10 to 100 nm, minifillers having an average particle size of from 0.01 to 15 µm, preferably 0.1 to 1 µm, as well as X-ray-opaque fillers having an average particle size of from 10 to 1000 nm, preferably 100 to 300 nm.

11. Use according to one of claims 1 to 10, **characterised in that** the dental material comprises
a) 0.1 to 70 wt %, in particular 1 to 60 wt %, preferably 5 to 50 wt % and particularly preferably 10 to 40 wt % of at least one compound of Formula I or I', in particular at least one compound of one of Formulae IA to ID,
b) 0.1 to 70 wt %, in particular 1 to 60 wt %, preferably 5 to 50 wt % and particularly preferably 10 to 40 wt % of at least one multifunctional azide, in particular at least one azide of Formula II,
c) 0 to 90 wt %, preferably 10 to 80 wt % and particularly preferably 20 to 70 wt % filler and
d) 0 to 70 wt %, preferably 1 to 50 wt % and particularly preferably 5 to 20 wt % solvent.

12. Use according to one of claims 1 to 11 for manufacturing inlays, onlays, crowns, bridges or veneering materials.

13. Use of at least one compound of Formula I or I' as defined in claim 1 or 2, preferably at least one compound of one of Formulae IA to ID as defined in claim 3 or 4, and in particular at least one azide of Formula II as defined in claim 5 or 6, for manufacturing a dental material and in particular a dental composite material.

## Revendications

1. Utilisation d'un matériau dentaire contenant au moins un composé de formule I ou I' : 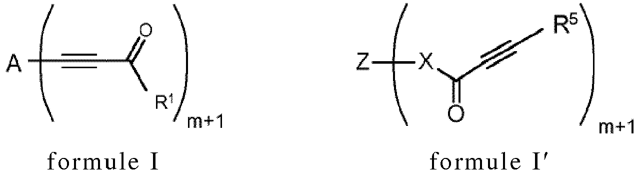 dans lesquelles formules
- A représente, indépendamment en chaque occurrence, un reste phénylène de valence m+1,
- R¹ représente, indépendamment en chaque occurrence,
- un reste aliphatique, alkyle en C₁-C₂₀, linéaire ou ramifié, qui peut être interrompu par un ou des chaînon(s) symbolisé(s) par -O- ou -S-,
- un reste phényle, qui peut porter un ou plusieurs substituant(s), de préférence choisi(s), indépendamment les uns des autres, dans l'ensemble formé par les groupes méthyle, éthyle, hydroxyle et méthoxy,
- ou un reste symbolisé par R³,
- R² représente, indépendamment en chaque occurrence,
- un atome d'hydrogène,
- un reste aliphatique, alkyle en C₁-C₂₀, linéaire ou ramifié,
qui peut être interrompu par un ou des chaînon(s) symbolisé(s) par -O- ou -S-,
- ou un reste phényle, qui peut porter un ou plusieurs substituant(s), de préférence choisi(s), indépendamment les uns des autres, dans l'ensemble formé par les groupes méthyle, éthyle, hydroxyle et méthoxy,
- R³ représente, indépendamment en chaque occurrence, un reste symbolisé par -Z-Y-R² ou par -Z-C( =O)-≡-A(R⁴)ₘ,
- R⁴ représente, indépendamment en chaque occurrence, un reste symbolisé par -≡-C(=O)-R³,
- R⁵ représente, indépendamment en chaque occurrence, un groupe phényle ou un reste symbolisé par R⁶,
- R⁶ représente, indépendamment en chaque occurrence, un reste symbolisé par -C(=O)-X-R ² ou par-C(=O)-X-Z(R⁷)ₘ,
- R⁷ représente, indépendamment en chaque occurrence, un reste symbolisé par -X-C(=O)-≡-R⁶,
- X représente, indépendamment en chaque occurrence, un chaînon symbolisé par O, S, NH ou CH₂, étant entendu que, si R⁵ représente un groupe phényle, ce chaînon représenté par X est absent,
- Y représente, indépendamment en chaque occurrence, un raccord symbolisé par -C(O)-O- ou -O-C(O)- ou un chaînon symbolisé par O ou S,
- Z représente
- un reste aliphatique, alcanediyle en C₁-C₂₀, linéaire ou ramifié, de valence m+1, qui peut être interrompu par un ou des chaînon(s) symbolisé(s) par -O- ou -S-,
- ou un reste phénylène, qui peut porter un ou plusieurs substituant(s), de préférence choisi(s), indépendamment les uns des autres, dans l'ensemble formé par les groupes méthyle, éthyle, hydroxyle et méthoxy,
- et l'indice m, indépendamment en chaque occurrence, vaut de 1 à 5, pour la fabrication de composites dentaires ou de matériaux de restauration dentaire.

2. Utilisation conforme à la revendication 1, pour laquelle, dans chacune des formules I et I', indépendamment l'une de l'autre :
- A représente, indépendamment en chaque occurrence, un reste phénylène de valence m+1,
- R¹ représente, indépendamment en chaque occurrence,
- un reste aliphatique, alkyle en C₁-C₁₀, linéaire ou ramifié, en particulier un groupe alkyle en C₁-C₆ et de préférence un groupe alkyle en C₁-C₃, qui peut être interrompu par un ou des chaînon(s) symbolisé(s) par -O- ou -S-,
- ou un reste symbolisé par R³,
- R² représente, indépendamment en chaque occurrence,
- un atome d'hydrogène,
- ou un reste aliphatique, alkyle en C₁-C₁₀, linéaire ou ramifié, en particulier un groupe alkyle en C₁-C₆ et de préférence un groupe alkyle en C₁-C₃, qui peut être interrompu par un ou des chaînon(s) symbolisé(s) par -O- ou -S-,
- R³ représente, indépendamment en chaque occurrence, un reste symbolisé par -Z-Y-R² ou par -Z-C(=O)-≡-A(R⁴)ₘ,
- R⁴ représente, indépendamment en chaque occurrence, un reste symbolisé par -≡-C(=O)-R³,
- R⁵ représente, indépendamment en chaque occurrence, un groupe phényle ou un reste symbolisé par R⁶,
- R⁶ représente, indépendamment en chaque occurrence, un reste symbolisé par -C(=O)-X-R ² ou par-C(=O)-X-Z(R⁷)ₘ,
- R⁷ représente, indépendamment en chaque occurrence, un reste symbolisé par -X-C(=O)-≡-R⁶,
- X représente, indépendamment en chaque occurrence, un chaînon symbolisé par O, S, NH ou CH₂, étant entendu que, si R⁵ représente un groupe phényle, ce chaînon représenté par X est absent,
- Y représente, indépendamment en chaque occurrence, un raccord symbolisé par -C(O)-O- ou -O-C(O)-,
- Z représente, indépendamment en chaque occurrence,
- un reste aliphatique, alcanediyle en C₁-C₁₀, linéaire ou ramifié, de valence m+1, en particulier un groupe alcanediyle en C₁-C₆ et de préférence un groupe alcanediyle en C₁-C₃, qui peut être interrompu par un ou des chaînon(s) symbolisé(s) par -O- ou -S-,
- ou un reste phénylène, qui peut porter un ou plusieurs substituant(s), de préférence choisi(s), indépendamment les uns des autres, dans l'ensemble formé par les groupes méthyle, éthyle, hydroxyle et méthoxy,
- et l'indice m, indépendamment en chaque occurrence, vaut 1, 2 ou 3.

3. Utilisation conforme à la revendication 1 ou 2, dans laquelle le matériau dentaire contient au moins un composé correspondant à l'une des formules IA à ID : 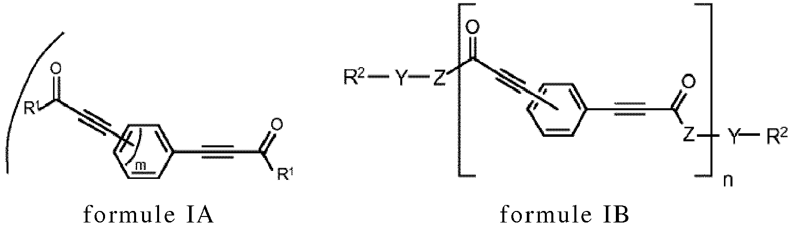 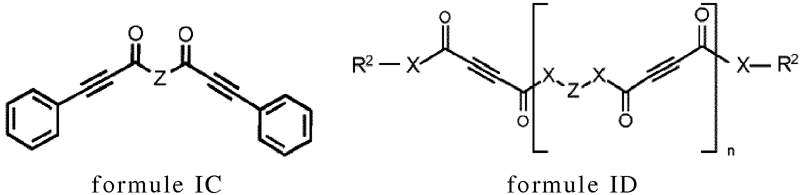 dans lesquelles formules
- R¹ représente, indépendamment en chaque occurrence,
- un reste aliphatique, alkyle en C₁-C₂₀, linéaire ou ramifié, qui peut être interrompu par un ou des chaînon(s) symbolisé(s) par -O- ou -S-,
- ou un reste phényle, qui peut porter un ou plusieurs substituant(s), de préférence choisi(s), indépendamment les uns des autres, dans l'ensemble formé par les groupes méthyle, éthyle, hydroxyle et méthoxy,
- R² représente, indépendamment en chaque occurrence,
- un atome d'hydrogène,
- un reste aliphatique, alkyle en C₁-C₂₀, linéaire ou ramifié, qui peut être interrompu par un ou des chaînon(s) symbolisé(s) par -O- ou -S-,
- ou un reste phényle, qui peut porter un ou plusieurs substituant(s), de préférence choisi(s), indépendamment les uns des autres, dans l'ensemble formé par les groupes méthyle, éthyle, hydroxyle et méthoxy,
- X représente, indépendamment en chaque occurrence, un chaînon symbolisé par O, S, NH ou CH₂,
- Y représente, indépendamment en chaque occurrence, un raccord symbolisé par -C(O)-O- ou -O-C(O)- ou un chaînon symbolisé par O ou S,
- Z représente
- un reste aliphatique, alcanediyle en C₁-C₂₀, linéaire ou ramifié, qui peut être interrompu par un ou des chaînon(s) symbolisé(s) par -O- ou -S-,
- ou un reste phénylène, qui peut porter un ou plusieurs substituant(s), de préférence choisi(s), indépendamment les uns des autres, dans l'ensemble formé par les groupes méthyle, éthyle, hydroxyle et méthoxy,
- l'indice m, indépendamment en chaque occurrence, vaut de 1 à 5,
- et l'indice n, indépendamment en chaque occurrence, vaut de 2 à 10.

4. Utilisation conforme à la revendication 3, pour laquelle, dans chacune des formules IA à ID, indépendamment l'une de l'autre :
- R¹ représente, indépendamment en chaque occurrence, un reste aliphatique, alkyle en C₁-C₁₀, linéaire ou ramifié, en particulier un groupe alkyle en C₁-C₆ et de préférence un groupe alkyle en C₁-C₃, qui peut être interrompu par un ou des chaînon(s) symbolisé(s) par -O- ou -S-,
- R² représente, indépendamment en chaque occurrence, un atome d'hydrogène ou un reste aliphatique, alkyle en C₁-C₁₀, linéaire ou ramifié, en particulier un groupe alkyle en C₁-C₆ et de préférence un groupe alkyle en C₁-C₃, qui peut être interrompu par un ou des chaînon(s) symbolisé(s) par -O- ou -S-,
- X représente, indépendamment en chaque occurrence, un chaînon symbolisé par O, S, NH ou CH₂,
- Y représente, indépendamment en chaque occurrence, un raccord symbolisé par -C(O)-O- ou -O-C(O)-,
- Z représente
- un reste aliphatique, alcanediyle en C₁-C₁₀, linéaire ou ramifié, en particulier un groupe alcanediyle en C₁-C₆ et de préférence un groupe alcanediyle en C₁-C₃, qui peut être interrompu par un ou des chaînon(s) symbolisé(s) par -O- ou -S-,
- ou un reste phénylène, qui peut porter un ou plusieurs substituant(s), de préférence choisi(s), indépendamment les uns des autres, dans l'ensemble formé par les groupes méthyle, éthyle, hydroxyle et méthoxy,
- l'indice m, indépendamment en chaque occurrence, vaut 1, 2 ou 3,
- et l'indice n, indépendamment en chaque occurrence, vaut de 3 à 8, et en particulier de 4 à 6.

5. Utilisation conforme à l'une des revendications 1 à 4, dans laquelle le matériau dentaire contient en outre au moins un azide de formule II :
A-[X-Q-(Y-N₃)ₙ]ₘ formule II
dans laquelle
- A représente un chaînon symbolisé par -O-, -N= ou -NR³ ou un raccord symbolisé par =N-N=, -NR³-N=, =N-NR³ ou -NR³-NR³-, ou un reste de valence m et de type alkylène, cycloalkylène, hétérocycloalkylène, arylène, hétéroarylène, mixte alkylène-cyclyle ou cyclyle-oxy-cyclyle, étant entendu
- que le terme « alkylène » désigne, indépendamment en chaque occurrence, un reste aliphatique en C₁-C₅₀, linéaire ou ramifié, qui peut être interrompu par un ou plusieurs chaînon(s), symbolisé(s) par -O-, -S- ou -NR³-, et/ou raccord(s) symbolisé(s) par -CO-O-, -O-CO-, -O-CO-O-, -CO-NR³-, -NR³-CO-, -O-CO-NR³-, -NR³-CO-O- ou -NR³-CO-NR³-,
- que le terme « cycloalkylène » désigne, indépendamment en chaque occurrence, un reste cycloaliphatique en C₃-C₁₈,
- que le terme « hétérocycloalkylène » désigne, indépendamment en chaque occurrence, un reste hétérocycloaliphatique en C₃-C₁₈,
- que le terme « arylène » désigne, indépendamment en chaque occurrence, un reste aromatique en C₆-C₁₈,
- que le terme « hétéroarylène » désigne, indépendamment en chaque occurrence, un reste hétéroaromatique en C₃-C₁₈,
- et que le terme « cyclyle » désigne, indépendamment en chaque occurrence, un reste cycloalkylène, hétérocycloalkylène, arylène ou hétéroarylène,
et que lesdits restes peuvent chacun porter, indépendamment, un ou plusieurs substituant(s), choisi(s) en particulier parmi les groupes alkyle en C₁-C₅, hydroxyle, méthoxy et acétyloxy,
- Q, indépendamment en chaque occurrence, ne représente rien ou représente un reste aliphatique, alkylène en C₁-C₂₀, linéaire ou ramifié, de valence n+1, qui peut être interrompu par un ou plusieurs chaînon(s) symbolisé(s) par -O- ou -S-, ou un reste phénylène,
étant entendu que lesdits restes peuvent chacun porter, indépendamment, un ou plusieurs substituant(s), choisi(s) en particulier parmi les groupes méthyle, éthyle, hydroxyle, méthoxy et acétyloxy,
- R³ représente, indépendamment en chaque occurrence, un atome d'hydrogène ou un groupe alkyle en C₁-C₁₀,
- X et Y, indépendamment en chaque occurrence, ne représentent rien ou représentent un chaînon symbolisé par -O-, -S-, -NR³- ou -CO- ou un raccord symbolisé par -CO-O-, -O-CO-, -O-CO-O-, -CO-NR³-, -NR³-CO-, -O-CO-NR³-, -NR³-CO-O- ou -NR³-CO-NR³-,
- l'indice m, indépendamment en chaque occurrence, vaut de 2 à 6,
- et l'indice n, indépendamment en chaque occurrence, vaut de 1 à 4.

6. Utilisation conforme à la revendication 5, dans laquelle, dans la formule II, indépendamment en chaque occurrence,
- A représente un chaînon symbolisé par -N=, ou un reste de valence m et de type alkylène, cycloalkylène, hétérocycloalkylène, arylène, hétéroarylène, mixte alkylène-cyclyle ou cyclyle-oxy-cyclyle, étant entendu
- que le terme « alkylène » désigne, indépendamment en chaque occurrence, un reste aliphatique en C₁-C₂₀, linéaire ou ramifié, qui peut être interrompu par un ou plusieurs chaînon(s) et/ou raccord(s) symbolisé(s) par -O-, -S-, -CO-O-, -O-CO- ou -O-CO-O-,
- que le terme « cycloalkylène » désigne, indépendamment en chaque occurrence, un reste cycloaliphatique en C₃-C₁₂,
- que le terme « hétérocycloalkylène » désigne, indépendamment en chaque occurrence, un reste hétérocycloaliphatique en C₃-C₁₂,
- que le terme « arylène » désigne, indépendamment en chaque occurrence, un reste aromatique en C₆-C₁₂,
- que le terme « hétéroarylène » désigne, indépendamment en chaque occurrence, un reste hétéroaromatique en C₃-C₁₂,
- et que le terme « cyclyle » désigne, indépendamment en chaque occurrence, un reste cycloalkylène, hétérocycloalkylène, arylène ou hétéroarylène,
et que lesdits restes peuvent chacun porter, indépendamment, un ou plusieurs substituant(s), choisi(s) en particulier parmi les groupes alkyle en C₁-C₃, méthoxy et acétyloxy,
- Q, indépendamment en chaque occurrence, ne représente rien ou représente un reste aliphatique, alkylène en C₁-C₁₀, linéaire ou ramifié, de valence n+1, qui peut être interrompu par un ou plusieurs chaînon(s) symbolisé(s) par -O- ou -S-, ou un reste phénylène,
étant entendu que lesdits restes peuvent chacun porter, indépendamment, un ou plusieurs substituant(s), choisi(s) en particulier parmi les groupes méthyle, éthyle, méthoxy et acétyloxy,
- R³ représente, indépendamment en chaque occurrence, un atome d'hydrogène ou un groupe alkyle en C₁-C₅, et en particulier, un groupe alkyle en C₁-C₃,
- X et Y, indépendamment en chaque occurrence, ne représentent rien ou représentent un chaînon symbolisé par -O- ou -S- ou un raccord symbolisé par -CO-O-, -O-CO-, -O-CO-O-, -CO-NR³-, -NR³⁻CO-, -O-CO-NR³-, -NR³-CO-O- ou -NR³-CO-NR³-,
- l'indice m, indépendamment en chaque occurrence, vaut 2, 3 ou 4,
- et l'indice n, indépendamment en chaque occurrence, vaut 1, 2 ou 3, en particulier 1 ou 2, et surtout 1.

7. Utilisation conforme à l'une des revendications 1 à 6, dans laquelle le matériau dentaire comprend un ou plusieurs monomère(s) polymérisable(s) par voie radicalaire.

8. Utilisation conforme à la revendication 7, dans laquelle le matériau dentaire comprend :
- l'un ou plusieurs des acrylate ou méthacrylate de méthyle, éthyle, hydroxy-éthyle, butyle, benzyle, tétrahydrofuryle ou isobornyle, méthacrylate de p-cumyl-phénoxy-éthylèneglycol (CMP-1E), di(méth)acrylate de bisphénol A, Bis-GMA, diméthacrylate de bisphénol A éthoxylé ou propoxylé, UDMA, di(méth)acrylate de diéthylèneglycol, triéthylèneglycol ou tétraéthylèneglycol, tri(méth)acrylate de triméthylolpropane, tétra(méth)acrylate de pentaérythritol, di(méth)acrylate ou tri(méth)acrylate de glycérol, di(méth)acrylate de butane-1,4-diol, di(méth)acrylate de décane-1,10-diol (D₃MA), di(méth)acrylate de docécane-1,12-diol,
- et/ou un ou plusieurs dérivé(s) d'acrylamide dont l'atome d'azote porte un ou deux substituant(s), N-éthyl-acrylamide, N,N-diméthyl-acrylamide, N-(2-hydroxy-éthyl)-acrylamide, N-(2-hydroxy-éthyl)-N-méthyl-acrylamide, un ou plusieurs dérivé(s) de méthacrylamide dont l'atome d'azote porte un substituant, N-éthyl-méthacrylamide, N-(2-hydroxy-éthyl)-méthacrylamide, N-vinyl-pyrrolidone, un ou plusieurs éther(s) d'allyle réticulant(s),
- et/ou un ou plusieurs dérivé(s) réticulant(s) de pyrrolidone, 1,6-bis(3-vinyl-pyrrolidone-2-yl)-hexane, un ou plusieurs bis(acrylamide) réticulant(s), méthylène-bis(acrylamide), éthylène-bis(acrylamide), un ou plusieurs bis(méthacrylamide) réticulant(s), N,N'-diéthyl-1,3-bis(acrylamido)-propane, 1,3-bis(méthacrylamido)-propane, 1,4-bis(acrylamido)-butane, 1,4-bis(acryloyl)-pipérazine, ou un mélange de tels composés.

9. Utilisation conforme à l'une des revendications 1 à 8, dans laquelle le matériau dentaire contient une charge organique et/ou inorganique.

10. Utilisation conforme à la revendication 9, dans laquelle le matériau dentaire contient au moins une charge inorganique en particules, choisie dans l'ensemble constitué par les matériaux amorphes à base d'oxydes ou d'oxydes mixtes, en particules de forme sphérique dont la taille moyenne vaut de 0,005 à 2 µm et de préférence de 0,1 à 1 µm, les charges dites « microfines », en nanoparticules dont la taille moyenne vaut de 5 à 200 nm et de préférence de 10 à 100 nm, les charges dites « minifines », en particules dont la taille moyenne vaut de 0,01 à 15 µm et de préférence de 0,1 à 1 µm, et les charges opaques aux rayons X, en particules dont la taille moyenne vaut de 10 à 1000 nm et de préférence de 100 à 300 nm.

11. Utilisation conforme à l'une des revendications 1 à 10, dans laquelle le matériau dentaire comprend :
a) de 0,1 à 70 % en poids, en particulier de 1 à 60 % en poids, de préférence de 5 à 50 % en poids, et mieux encore de 10 à 40 % en poids, d'au moins un composé de formule I ou I', et en particulier, d'au moins un composé de l'une des formules IA à ID,
b) de 0,1 à 70 % en poids, en particulier de 1 à 60 % en poids, de préférence de 5 à 50 % en poids, et mieux encore de 10 à 40 % en poids, d'au moins un azide polyfonctionnel, en particulier d'au moins un azide de formule II,
c) de 0 à 90 % en poids, de préférence de 10 à 80 % en poids, et mieux encore de 20 à 70 % en poids, d'une charge,
d) et de 0 à 70 % en poids, de préférence de 1 à 50 % en poids, et mieux encore de 5 à 20 % en poids, d'un solvant.

12. Utilisation conforme à l'une des revendications 1 à 11, pour la fabrication de prothèses inlay ou onlay, de couronnes, de bridges ou de placages.

13. Utilisation d'au moins un composé de formule I ou I', tel que défini dans la revendication 1 ou 2, de préférence d'au moins un composé de l'une des formules IA à ID, tel que défini dans la revendication 3 ou 4, et en particulier d'au moins un azide de formule II, tel que défini dans la revendication 5 ou 6, pour la fabrication d'un matériau dentaire et en particulier d'un matériau composite dentaire.
